# EUROPEAN PATENT APPLICATION

(11) **EP 3 932 948 A1**
(43) Date of publication of application: **05.01.2022**
(21) Application number: 20762071.7
(22) Date of filing: 20.02.2020
(51) Int. Cl.: C07K 16/28, C12N 15/13, C12N 15/63, C12P 21/08, A61K 39/395, A61P 35/00

(54) **ANTIBODY BINDING HUMAN LAG-3, AND PREPARATION METHOD AND USE THEREOF**

(30) Priority: 28.02.2019 CN 201910148914
(71) Applicant: Sunshine Guojian Pharmaceutical (Shanghai) Co., Ltd., China (Shanghai) Pilot Free Trade Zone Shanghai 201203 (CN)
(72) Inventor: ZHAO, Jie, Shanghai 201203 (CN); HUANG, Haomin, Shanghai 201203 (CN); ZHU, Zhenping, Shanghai 201203 (CN)
(74) Representative: Plougmann Vingtoft a/s
(86) International application number: PCT/CN2020/076023
(87) International publication number: WO 2020/173378

(57) **Abstract**

Provided are an antibody capable of specifically binding human LAG-3 or an antigen binding fragment thereof. The antibody or antigen binding fragment thereof has a biological activity for enhancing a mixed lymphocyte reaction, and can be used for preparing a drug for cancer immunotherapy.

## Description

### Field of the Invention

The present invention relates to the field of antibodies. More particularly,, the present invention discloses an antibody that binds to human LAG-3, its preparation method and use thereof.

### Background of the Invention

Various types of malignant tumors have now become the main killers of human beings, and the incidence rate is increasing year by year. Although conventional surgery, chemotherapy and radiotherapy have certain therapeutic effects, they often bring huge damage and toxic side effects to the patient's body. Though current targeted monoclonal antibody drugs (for example, trastuzumab targeting HER2) and targeted small molecule kinase inhibitors (for example, imatinib targeting certain tyrosine kinases) have achieved gratifying clinical effects in tumor treatment, these drugs are only effective for tumors that express specific targets, and may be used to treat limited types of cancer, and often have a low objective response rate. The application of such antibodies is prone to develop drug resistance in the tumors, which thus recur and progress again. Tumor immunotherapy refers to the application of immunological principles and methods to activate the immune system, break the immune system's immune tolerance to tumors, enhance the recognition of tumor antigens by immune cells, stimulate and enhance the body's anti-tumor immune response, so that the body's immune system suppresses and kills tumor cells, and finally achieve the goal of treating tumors. Tumor immunotherapy has attracted much attention recently and is the focus of the field of tumor treatment. In recent years, great news of tumor immunotherapy has continued to emerge. At present, programmed death receptor 1 (PD-1) is undoubtedly the most dazzling star target in cancer immunotherapy, and immune checkpoint inhibitors that target it, such as nivolumab and pembrolizumab and other monoclonal antibodies, have demonstrated powerful anti-tumor activity in the clinical treatment of some tumor types such as melanoma, non-small cell lung cancer, etc., and have been approved by the US Food and Drug Administration (FDA) for clinical application. Tumor immunotherapy was named the most important scientific breakthrough of the Year 2013 by Science magazine due to its excellent efficacy and innovation. Tumor immunotherapy is expected to become a revolution in the field of tumor treatment following surgery, chemotherapy, radiotherapy and targeted therapy.

T cells play an important role in the immune system, but the activation of T cells depends on antigen-presenting cells digesting harmful foreign antigens and re-presenting them into an antigen type that can be recognized by T cells. There are a group of proteins involved in regulation on T cells and antigen-presenting cells, which have the function of assisting in regulating the signal transduction of T cell receptors. These accessory receptors are divided into two categories, one is co-stimulatory receptors that are responsible for transmitting activation signals, and the other is co-inhibitory receptors that transmit inhibitory signals. These inhibitory molecules are now called immune checkpoint receptors and ligands. The star molecule PD-1 mentioned above is a typical immune checkpoint receptor, a member of the CD28 superfamily, and its important ligand is programmed death ligand 1 (PD-L1). The binding of PD-1 and PD-L1 mediates the co-inhibitory signal of T cell activation and negatively regulates T cell activation and proliferation. In addition, Professor Chen Lieping first discovered that PD-L1 is highly expressed in tumor tissues and regulates the function of tumor-infiltrating CD8+ T cells. At present, immunomodulatory therapy targeting PD-1/PD-L1 has achieved great success in clinical anti-tumor therapy.

Lymphocyte activation gene-3 (LAG-3) is mainly expressed in activated T lymphocytes and is a negative immunoregulatory molecule similar to PD-1. The main ligand of LAG-3 is the MHC II molecular family, and it can compete with CD4 molecules to bind to MHC II ligands with higher affinity, and transduce inhibitory signals into cells, thereby negatively regulating cell activation, proliferation and dynamic balance, with a molecular mechanism very similar to PD-1. LAG-3 can help T cells maintain a state of immune tolerance, or drive continuously activated T cells into a state of "exhaustion", and has the functions of maintaining homeostasis and participating in negative immune regulation, and is closely related to the occurrence and development of tumors. At present, some preliminary studies have shown that antibodies that block LAG-3 can enhance the immune response, and the combination with PD-1 antibody can play a significant synergistic activation effect, and has great potential in the field of cancer immunotherapy.

At present, most of the international research and development of monoclonal antibody drugs targeting LAG-3 is still in the early stage. The main research and development companies are international pharmaceutical giants such as Bristol-Myers Squibb (BMS) and Novartis. Among them, Bristol-Myers Squibb's anti-LAG-3 monoclonal antibody BMS-986016 is in the first phase of clinical research, the main research objective is the safety and effectiveness of its combination with the PD-1 antibody nivolumab, and the research results show that the combination of BMS-986016 and nivolumab has good efficacy in the clinical treatment of melanoma. In addition, anti-LAG-3 antibodies can also be used to treat autoimmune diseases. The basic mechanism is to kill activated T cells through antibody-dependent cell-mediated cytotoxicity (ADCC), thereby achieving the purpose of inhibiting the immune response and treating autoimmune diseases. However, there is still an urgent need to develop more new, specific and efficient drugs targeting LAG-3 for clinical applications of immunotherapy.

### Summary of the Invention

In order to solve the above technical problems, the inventors of the present invention conducted a large number of experiments, from antigen immunization, hybridoma screening, antibody expression and purification until biological activity identification, screened and obtained two murine antibodies No. 134 and 2-34 (i.e., 5E7) that specifically binds to human LAG-3. On this basis, their chimeric antibodies 134-Chimeric, 5E7-Chimeric and humanized antibodies 134-Hu-IgG4-C91S and 5E7-Hu-IgG4 were further constructed. The studies of the present invention show that the murine antibodies No. 134 and 2-34 (i.e., 5E7) have a new epitope that binds to human LAG-3. Cell-level experiments showed that the humanized antibodies 134-Hu-IgG4-C91S and 5E7-Hu-IgG4 can effectively block the binding of LAG-3 to Raji cells and enhance the secretion of IL-2 by SEA-stimulated PBMC. Therefore, the antibody or antigen-binding fragment thereof that binds to human LAG-3 developed by the present invention can be used to prepare a drug for cancer immunotherapy.

Thus, the first object of the present invention is to provide an antibody or antigen-binding fragment thereof that binds to human LAG-3.

The second object of the present invention is to provide another antibody or antigen-binding fragment thereof that binds to human LAG-3.

The third object of the present invention is to provide a nucleotide sequence encoding the antibody or antigen-binding fragment thereof that binds to human LAG-3.

The fourth object of the present invention is to provide an expression vector comprising the nucleotide sequence.

The fifth object of the present invention is to provide a host cell comprising the expression vector.

The sixth object of the present invention is to provide a method of preparing the antibody or antigen-binding fragment thereof that binds to human LAG-3.

The seventh object of the present invention is to provide a pharmaceutical composition comprising the antibody or antigen-binding fragment thereof that binds to human LAG-3.

The eighth object of the present invention is to provide the use of the antibody or antigen-binding fragment thereof that binds to human LAG-3, or of the pharmaceutical composition.

In order to achieve the above objects, the present invention adopts the following technical solutions:
The first aspect of the present invention provides an antibody or antigen-binding fragment thereof that binds to human LAG-3, and the human LAG-3 epitope to which it binds comprises the following amino acid sequence: AAAPGHPLA (SEQ ID NO: 50).

According to a preferred embodiment of the present invention, the human LAG-3 epitope to which it binds comprises the following amino acid sequence: GPPAAAPGHPLA (SEQ ID NO: 48) or AAAPGHPLAPGPHPAAPSS (SEQ ID NO: 49).

The second aspect of the present invention provides an antibody or antigen-binding fragment thereof that binds to human LAG-3, comprising:
(a) heavy chain complementarity-determining regions HCDR1, HCDR2, HCDR3, the HCDR1 having the amino acid sequence as shown in SEQ ID NO: 9, the HCDR2 having the amino acid sequence as shown in SEQ ID NO: 10, and the HCDR3 having the amino acid sequence as shown in SEQ ID NO: 11, and light chain complementarity determining regions LCDR1, LCDR2, LCDR3, the LCDR1 having the amino acid sequence as shown in SEQ ID NO: 12, the LCDR2 having the amino acid sequence as shown in SEQ ID NO: 13, and the LCDR3 having the amino acid sequence as shown in SEQ ID NO: 14 or SEQ ID NO: 43; or
(b) heavy chain complementarity-determining regions HCDR1, HCDR2, HCDR3, the HCDR1 having the amino acid sequence as shown in SEQ ID NO: 15, the HCDR2 having the amino acid sequence as shown in SEQ ID NO: 16, and the HCDR3 having the amino acid sequence as shown in SEQ ID NO: 17, and light chain complementarity-determining regions LCDR1, LCDR2, LCDR3, the LCDR1 having the amino acid sequence as shown in SEQ ID NO: 18, the LCDR2 having the amino acid sequence as shown in SEQ ID NO: 19, and the LCDR3 having the amino acid sequence as shown in SEQ ID NO: 20.

According to the present invention, the antibody is a monoclonal antibody or a polyclonal antibody. Preferably, the antibody is a monoclonal antibody.

According to the present invention, the antibody is a murine antibody, a chimeric antibody, or a humanized antibody. Preferably, the antibody is a humanized antibody

According to the present invention, the antigen-binding fragment comprises a Fab fragment, a F(ab)'2 fragment, a Fv fragment, etc.

According to a preferred embodiment of the present invention, the antibody or antigen-binding fragment thereof that binds to human LAG-3 comprises a heavy chain variable region and a light chain variable region, selected from the group consisting of:
(a) a heavy chain variable region having the amino acid sequence as shown in SEQ ID NO: 2, and a light chain variable region having the amino acid sequence as shown in SEQ ID NO: 4;
(b) a heavy chain variable region having the amino acid sequence as shown in SEQ ID NO: 6, and a light chain variable region having the amino acid sequence as shown in SEQ ID NO: 8;
(c) a heavy chain variable region having the amino acid sequence as shown in SEQ ID NO: 22, and a light chain variable region having the amino acid sequence as shown in SEQ ID NO: 24; and
(d) a heavy chain variable region having the amino acid sequence as shown in SEQ ID NO: 26, and a light chain variable region having the amino acid sequence as shown in SEQ ID NO: 28.

According to a preferred embodiment of the present invention, the antibody or antigen-binding fragment thereof that binds to human LAG-3 comprises a heavy chain constant region and a light chain constant region. The heavy chain constant region is selected from the group consisting of IgG1, IgG2, IgG3, and IgG4 heavy chain constant region, and the light chain constant region is selected from κ or λ light chain constant region. Preferably, the heavy chain constant region is IgG4 heavy chain constant region, and the light chain constant region is κ light chain constant region. More preferably, the heavy chain constant region has the amino acid sequence as shown in SEQ ID NO: 30, the light chain constant region has the amino acid sequence as shown in SEQ ID NO: 34.

According to a preferred embodiment of the present invention, the antibody or antigen-binding fragment thereof that binds to human LAG-3 comprises a heavy chain and a light chain, selected from the group consisting of:
(a) a heavy chain having the amino acid sequence as shown in SEQ ID NO: 32, and a light chain having the amino acid sequence as shown in SEQ ID NO: 36; and
(b) a heavy chain having the amino acid sequence as shown in SEQ ID NO: 38, and a light chain having the amino acid sequence as shown in SEQ ID NO: 40.

The third aspect of the present invention provides a nucleotide sequence, which encodes the antibody or antigen-binding fragment thereof that binds to human LAG-3 as described in any one of the above.

According to a preferred embodiment of the present invention, the nucleotide sequence comprises:
(a) a nucleotide sequence encoding the heavy chain variable region as shown in SEQ ID NO: 1, and a nucleotide sequence encoding the light chain variable region as shown in SEQ ID NO: 3;
(b) a nucleotide sequence encoding the heavy chain variable region as shown in SEQ ID NO: 5, and the nucleotide sequence encoding the light chain variable region as shown in SEQ ID NO: 7;
(c) a nucleotide sequence encoding the heavy chain variable region as shown in SEQ ID NO: 21, and a nucleotide sequence encoding the light chain variable region as shown in SEQ ID NO: 23; or
(d) a nucleotide sequence encoding the heavy chain variable region as shown in SEQ ID NO: 25, and a nucleotide sequence encoding the light chain variable region as shown in SEQ ID NO: 27.

According to a preferred embodiment of the present invention, the nucleotide sequence comprises a nucleotide sequence encoding the heavy chain constant region as shown in SEQ ID NO: 29, and a nucleotide sequence encoding the light chain constant region as shown in SEQ ID NO: 33.

According to a preferred embodiment of the present invention, the nucleotide sequence comprises:
(a) a nucleotide sequence encoding the heavy chain as shown in SEQ ID NO: 31, and a nucleotide sequence encoding the light chain as shown in SEQ ID NO: 35; or
(b) a nucleotide sequence encoding the heavy chain as shown in SEQ ID NO: 37, and a nucleotide sequence encoding the light chain as shown in SEQ ID NO: 39.

The fourth aspect of the present invention provides an expression vector, which comprises the nucleotide sequence as described in any one of the above.

The fifth aspect of the present invention provides a host cell, which comprises the expression vector as described above.

The sixth aspect of the present invention provides a method of preparing an antibody or antigen-binding fragment thereof that binds to human LAG-3 as described above, which comprises the following steps:
a) under expression conditions, culturing the host cell described above, to express the antibody or antigen-binding fragment thereof that binds to human LAG-3;
b) isolating and purifying the antibody or antigen-binding fragment thereof that binds to human LAG-3 of step a).

The seventh aspect of the present invention provides a pharmaceutical composition, which comprises the antibody or antigen-binding fragment thereof that binds to human LAG-3 as described in any one of the above, and a pharmaceutically acceptable carrier.

According to a preferred embodiment of the present invention, the pharmaceutical composition further comprises a PD-1 inhibitor. Preferably, the PD-1 inhibitor is an antibody or antigen-binding fragment thereof that binds to PD-1.

The eighth aspect of the present invention provides the use of the antibody or antigen-binding fragment thereof that binds to human LAG-3 as described in any one of the above, or the pharmaceutical composition as described in any one of the above, in the preparation of a drug for the treatment of cancers.

According to the present invention, the cancer is selected from the group consisting of melanoma, renal cell carcinoma, non-small cell lung cancer, classic Hodgkin's lymphoma, urothelial carcinoma, colorectal cancer, liver cancer, etc.

### Beneficial effects:

The present invention discloses an antibody or antigen-binding fragment thereof capable of specifically binding to human LAG-3, which has a novel epitope that binds to human LAG-3 and a good biological activity of enhancing mixed lymphocyte reaction, and can be applied to prepare drugs for cancer immunotherapy, and has good clinical application prospects.

### Description of the Drawings

Fig. 1 shows the effect of murine anti-human LAG-3 antibody on the mixed lymphocyte reaction, where Fig. 1A is the first round of screening, and Fig. 1B is the second round of screening.
Fig. 2 shows the comparison of the relative affinity between the humanized antibody and the chimeric antibody of anti-human LAG-3 antibody.
Fig. 3 shows the enhanced effect of humanized anti-human LAG-3 antibody on the secretion of IL-2 by PBMC, where the PBMCs in Figs. 3A and 3B are from different donors.
Fig. 4 shows the binding of humanized anti-human LAG-3 antibody to LAG-3 on the cell surface.
Fig. 5 shows a schematic diagram of epitope of the anti-human LAG-3 antibody.
Fig. 6 shows the blocking effect of humanized anti-human LAG-3 antibody on the binding of LAG-3 to Raji cells.
Fig. 7 shows the blocking effect of anti-human LAG-3 antibody on the binding of FGL1 to LAG-3 detected by flow cytometry.
Fig. 8 shows the inhibitory effect of anti-human LAG-3 antibody on the MC38 xenograft model in transgenic mice.

### Detailed Description of the Invention

In the present invention, the term "LAG-3" refers to lymphocyte activation gene-3, also known as CD223.

In the present invention, the terms "antibody (Ab)" and "immunoglobulin G (IgG)" are heterotetrameric glycoproteins of about 150,000 daltons with identical structural characteristics, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable region (VH) followed by constant regions. The heavy chain constant region is composed of three structural domains, CH1, CH2, and CH3. Each light chain has a variable region (VL) at one end and a constant region at its other end, the light chain constant region includes a domain CL; the constant region of the light chain is aligned with the first constant region of the heavy chain, and the light chain variable region is aligned with the variable region of the heavy chain. The constant regions are not involved directly in binding an antibody to an antigen, but they exhibit various effector functions, such as participation in antibody-dependent cell-mediated cytotoxicity (ADCC). The heavy chain constant region comprises IgG1, IgG2, IgG3, IgG4, IgM, IgA1, IgA2, IgD and IgE; the light chain constant region comprises κ (Kappa) or λ (Lambda).

The antibody of the present invention comprises monoclonal antibodies, polyclonal antibodies, multispecific antibodies formed from at least two antibodies (such as bispecific antibodies), etc.

In the present invention, the term "monoclonal antibody (mAb)" refers to an antibody obtained from a population of substantially homogeneous antibodies, that is, the individual antibodies contained in the population are the same, except for a few possible naturally occurring mutations. Monoclonal antibodies target a single antigen site with high specificity. Moreover, unlike conventional polyclonal antibody preparations (usually with different antibodies directed against different determinants), each monoclonal antibody is directed against a single determinant on the antigen. In addition to their specificity, the benefit of monoclonal antibodies is that they are synthesized by hybridoma culture and are not contaminated by other immunoglobulins. The modifier "monoclonal" indicates the characteristics of an antibody, which is obtained from a substantially uniform antibody population, and should not be interpreted as requiring any special method to produce the antibody.

In the present invention, the term "antigen-binding fragment" refers to a fragment of an antibody capable of specifically binding to an antigen (such as human LAG-3). Examples of the antigen-binding fragments of the present invention include Fab fragments, F(ab')2 fragments, Fv fragments, etc. A Fab fragment is a fragment produced by digesting an antibody with papain. A F(ab')2 fragment is a fragment produced by digesting an antibody with pepsin. A Fv fragment is composed of dimers in which the heavy chain variable region and the light chain variable region of an antibody are closely and non-covalently linked.

In the present invention, the term "Fc segment" means that papain can cleave an antibody into two identical Fab segments and one Fc segment. Fc segment, namely fragment crystallizable (Fc), is composed of the CH2 and CH3 domains of the antibody. Fc segment has no antigen binding activity and is the site where the antibody interacts with effector molecules or cells.

In the present invention, the term "variable" refers to the fact that certain portions of the variable regions differ extensively in sequence among antibodies and are responsible for the binding specificity of each particular antibody for its particular antigen. However, the variability is not evenly distributed through the variable regions of antibodies. It is concentrated in three segments called complementarity-determining regions (CDRs) or hypervariable regions both in the light chain variable regions and the heavy chain variable regions. The more highly conserved portions of the variable regions are called the framework regions (FR). The variable regions of native heavy and light chains each comprise four FR regions, largely adopting a β-sheet configuration, connected by three CDRs, which form loops connecting, and in some cases forming part of the β-sheet structure. The CDRs in each chain are held together in close proximity by the FR regions and, with the CDRs from the other chain, contribute to the formation of the antigen binding site of antibodies (see Kabat et al., NIH Publ.No.91-3242, Volume I, Pages 647-669 (1991)).

The antibody of the present invention comprises murine antibodies, chimeric antibodies, humanized antibodies, etc.

In the present invention, the term "murine antibody" refers to an antibody derived from rats or mice, preferably mice. The murine antibody of the present invention is obtained by immunizing mice with the extracellular domain of human LAG-3 as an antigen and screening hybridoma cells. More preferably, the murine antibody of the present invention comprises antibodies No. 134 and No. 2-34 (i.e., 5E7).

In the present invention, the term "chimeric antibody" refers to an antibody that comprises heavy and light chain variable region sequences from one species and constant region sequences from another species, such as an antibody having mouse heavy and light chain variable regions linked to human constant region. Preferably, the chimeric antibody of the present invention is obtained by splicing the heavy chain variable region sequence and the light chain variable region sequence of the murine antibodies No. 134 and No. 2-34 (i.e., 5E7) with the human constant region. More preferably, the heavy chain of the chimeric antibody of the present invention is obtained by splicing the heavy chain variable region sequence of the murine antibodies No. 134 and No. 2-34 (i.e., 5E7) with human IgG4 heavy chain constant region, respectively, and the light chain is obtained by splicing the light chain variable region sequence of the murine antibodies No. 134 and No. 2-34 (i.e., 5E7) with human Kappa light chain constant region, respectively. Most preferably, the chimeric antibody of the present invention comprises 134-Chimeric and 5E7-Chimeric.

In the present invention, the term "humanized antibody" means that the CDRs are derived from a non-human (preferably, mouse) antibody, while the remaining parts (including framework regions and constant regions) are derived from human antibody. In addition, framework region residues may be altered to preserve the binding affinity. Preferably, the humanized antibody of the present invention is obtained by recombining the CDR regions of the murine antibody No. 134 and No. 2-34 (i.e., 5E7) and the non-CDR regions derived from a human antibody, and subjecting the embedded residues, the residues that directly interact with the CDR region, and the residues that have important influence on the conformation of VL and VH of the murine antibodies No. 134 and No. 2-34 (i.e., 5E7) to back mutation. More preferably, the humanized antibody of the present invention comprises 134-Hu-IgG4-C91S and 5E7-Hu-IgG4.

In the present invention, the terms "epitope" and "human LAG-3 epitope" refer to regions located on human LAG-3 and specifically binding to an antibody. Preferably, the human LAG-3 epitope of the present invention is located in the extracellular domain of human LAG-3, and the extracellular domain of human LAG-3 comprises the amino acid sequence as shown in SEQ ID NO: 41. More preferably, the human LAG-3 epitope of the present invention comprises the following amino acid sequence: AAAPGHPLA (SEQ ID NO: 50). Most preferably, the human LAG-3 epitope of the present invention comprises the following amino acid sequence: GPPAAAPGHPLA (SEQ ID NO: 48) or AAAPGHPLAPGPHPAAPSS (SEQ ID NO: 49).

In the present invention, the term "antibody that binds to human LAG-3" or "anti-human LAG-3 antibody" refers to an antibody that specifically binds to an epitope of human LAG-3. The term "specifically bind" means that the antibody binds to antigen with an equilibrium dissociation constant (KD) of less than 1×10⁻⁷ M or less, preferably 1×10⁻⁸ M or less, more preferably 1×10⁻⁹ M or less, 1×10⁻¹⁰ M or more, 1×10⁻¹¹ M or less. In the present invention, the term "KD" refers to the equilibrium dissociation constant of a specific antibody-antigen interaction, which is used to describe the binding affinity between antibody and antigen. The smaller the equilibrium dissociation constant is, the tighter the antibody-antigen binding is, and the higher the affinity between the antibody and the antigen is. For example, surface plasmon resonance (abbreviated as SPR) is used to measure the binding affinity of antibody to antigen in BIACORE instrument or ELISA is used to measure the relative binding affinity of antibody to antigen.

In the present invention, the term "expression vector" may be pTT5, pSECtag series, pCGS3 series, pCDNA series vectors, as well as other vectors used in mammalian expression systems, etc. The expression vector comprises a fusion DNA sequence connected with appropriate transcription and translation regulatory sequences.

In the present invention, the term "host cell" refers to a cell suitable for expressing the expression vector as described above, which may be a eukaryotic cell, for example, mammalian or insect host cell culture system may be used to express the fusion protein of the present invention, CHO (Chinese hamster Ovary), HEK293, COS, BHK, as well as derived cells of the above-mentioned cells are applicable to the present invention.

In the present invention, the term "pharmaceutical composition" means that the antibody or antigen-binding fragment thereof that binds to human LAG-3 of the present invention can be combined with a pharmaceutically acceptable carrier to form a pharmaceutical preparation composition, so as to exert a therapeutic effect more stably. These preparations can ensure the conformational integrity of the amino acid core sequences of the antibody or antigen-binding fragment thereof that binds to human LAG-3 disclosed in the present invention, and meanwhile, protect the multifunctional groups of the protein from degradation (including but not limited to aggregation, deamination or oxidation).

The following examples are used to further illustrate the present invention and should not be construed as limiting the present invention. The examples do not include a detailed description of traditional methods, such as those methods of constructing expression vectors and preparing plasmids, methods of inserting genes encoding proteins into such vectors and plasmids, or methods of transfecting plasmids into host cells. Such methods are well known to those of ordinary skill in the art, and are described in many publications, including Sambrook, J., Fritsch, E.F. and Maniais, T. (1989) Molecular Cloning: A Laboratory Manual, 2nd edition, Cold spring Harbor Laboratory Press.

### Example 1. Preparation of human LAG-3 protein and anti-LAG-3 positive control antibody

The amino acid sequence of the extracellular domain of human LAG-3 was from https://www.uniprot.org/uniprot/P18627, and the amino acid sequence is as shown in SEQ ID NO: 41, which was subjected to codon optimization and gene synthesis by Sangon Biotech (Shanghai) Co.,Ltd., and then the synthesized gene fragments were cloned into the pUC57 vector. Primers were designed and used to amplify the extracellular domain (ECD) coding region (SEQ ID NO: 41) of LAG-3 and the Fc coding region (SEQ ID NO: 51) of human immunoglobulin IgG1 by PCR (Polymerase Chain Reaction). The above amplified fragments were recovered and spliced together by recombination PCR, and meanwhile, the signal peptide coding region (MGVKVLFALICIAVAEA, SEQ ID NO: 42) was introduced, and the corresponding restriction sites were introduced at both ends: the pTT5 expression vector (purchased from NRC Biotechnology Research Institute) and the above recombinant PCR fragment were cut with restriction endonucleases; the two groups of enzyme-digested products were purified, ligated with a ligase, and transformed into E. coli TOP10 competent cells, and plated on LB (Amp-resistant) medium for culture overnight. Monoclonal colonies were picked, cultured and amplified, and then the plasmid was extracted, and cut with restriction endonucleases to identify whether the target gene fragments were inserted. The qualified positive clones were selected for sequencing. The clones with the correct sequence were selected for amplification, and then the plasmids were extracted. The constructed LAG-3 expression vector was transferred into HEK293E cells (purchased from NRC Biotechnology Research Institute) for expression using polyethyleneimine (PEI). After 5 days, the cell supernatant was collected and purified by Protein A affinity chromatography. The resulting protein was named LAG-3-ECD-hFc, which was used for immunization of mice and subsequent screening processes. HEK293E cells were cultured in FreeStyle 293 Expression Medium (purchased from Thermo Fisher Scientific), a serum-free, chemically defined medium. Protein quantification was carried out by ultraviolet spectrophotometry.

The amino acid sequences of the heavy chain variable region and the light chain variable region of anti-LAG-3 monoclonal antibody that was used as a positive control were from SEQ ID NOs: 12 and 14 in the US patent application US20140093511A1, which were subjected to codon optimization and gene synthesis by Sangon Biotech (Shanghai) Co., Ltd.. The heavy chain variable region and light chain variable region genes were linked to the human IgG4 heavy chain constant region (the nucleotide sequence is shown in SEQ ID NO: 29, and the amino acid sequence is shown in SEQ ID NO: 30) and human Kappa light chain constant region (the nucleotide sequence is shown in SEQ ID NO: 33, and the amino acid sequence is shown in SEQ ID NO: 34), respectively, and the signal peptide sequence (SEQ ID NO: 42) was introduced to construct complete heavy chain and light chain genes, and then according to a similar method as described above, the complete heavy chain and light chain genes were cloned into the pTT5 expression vector, respectively. According to the same method, the pTT5 expression vector containing heavy chain and light chain genes was combined and transferred into HEK293E cells for expression; after 5 days, the cell supernatant was collected and the antibody was purified by Protein A affinity chromatography. The resulting antibody was named Anti-LAG3.5. HEK293E cells were cultured in FreeStyle 293 Expression Medium (purchased from Thermo Fisher Scientific), a serum-free, chemically defined medium. Protein quantification was carried out by ultraviolet spectrophotometry.

### Example 2. Immunization of mice with human LAG-3-ECD-hFc as an antigen and preparation and screening of hybridomas

LAG-3-ECD-hFc prepared in Example 1 was diluted with normal saline to an appropriate concentration, mixed with an equal volume of Freund's complete adjuvant (purchased from Sigma), phaco-emulsified, and then administrated to 4-5 weeks old Balb/c mice (purchased from Shanghai Lingchang Biotechnology Co., Ltd., animal production license number: SCXK (Shanghai) 2013-0018) by multipoint subcutaneous injection; after three weeks, LAG-3-ECD-hFc was diluted with normal saline and mixed with an equal volume of Freund's incomplete adjuvant (purchased from Sigma), fully phaco-emulsified, and then administrated to mice by multipoint subcutaneous immunization; such steps were repeated after two weeks. On day 7 after the third immunization, a small amount of blood was collected from each mouse to separate serum, and serum titer was determined by conventional ELISA. The mice with serum antibody titer> 10,000 were selected and each mouse was injected with 10 µg antigen protein by tail vein after seven days.

On day 3 after intravenous injection of antigen protein, some mice were selected, sacrificed and dissected to remove the spleen. After grinding, the spleen cells were collected, counted, and used to prepare hybridomas. Herein, myeloma Sp2/0 cells (from the cell bank of the Type Culture Collection Committee of the Chinese Academy of Sciences) were selected as a cell fusion ligand. Sp2/0 cells were cultured in a cell incubator (37°C, 5% CO₂). The medium used was RPMI-1640 complete medium (with the preparation method as follows: RPMI-1640 basic medium was supplemented with 10% Fetal bovine serum and 1% Penicillin-Streptomycin, the above materials were purchased from Thermo Fisher Scientific), the medium was changed one day before the cell fusion, so that the cells were in a better growth state. Herein, the electrofusion method was used to prepare hybridoma cells. The electrofusion method and hybridoma screening process are as follows: the two cells were mixed according to the ratio of spleen cells: Sp2/0 cells = 2:1, centrifuged and the supernatant was discarded; then the cells were washed three times with cell fusion buffer (purchased from BTX); the cell pellet was suspended at a density of 1×10⁷ /ml in cell fusion buffer; 2 ml of cell suspension was added to the cell fusion pool, placed on the electrofusion instrument ECM2001 (purchased from BTX), and processed under certain conditions (AC 60V, 30S; DC 1700 V, 40µS, 3X; POST AC 60V, 3S); and then, the treated cells were gently transferred to a RPMI-1640 complete medium preheated at 37°C and placed at room temperature for 1 hour; the treated cells were inoculated into 96-well plates at a density of 10⁴/well, 100 µl per well; the next day, each well was supplemented with 100 µl of RPMI-1640 complete medium containing 2×HAT (50×HAT purchased from Thermo Fisher Scientific, appropriately diluted with culture medium as needed before use); the culture medium was replaced with fresh RPMI-1640 complete medium containing 1×HAT on days 4 and 7; samples were taken for ELISA detection on day 9 after fusion to identify positive clones.

The detection of positive clones (clones capable of secreting anti-LAG-3 antibody) was carried out by ELISA: the microplates were coated with LAG-3-ECD-hFc antigen with a coating volume of 100 ng/well; incubated at room temperature for 2 hours and washed with PBST (PBS containing 0.05% Tween-20); each well was blocked with 200 µl of PBST solution containing 1% Bovine Serum Albumin (BSA), and incubated at room temperature 1 hour; washed with PBST, pat dried, and stored in a refrigerator for later use. During the detection, hybridoma culture supernatant was added to the microplates at 100 µl/well, incubated for about 1 hour; washed with PBST 3 times; the HRP-Goat-Anti-Mouse IgG antibody (purchased from KPL) was appropriately diluted with PBST containing 1% BSA, and incubated at room temperature for about 1 hour; washed with PBST 3 times; 100 µl of chromogenic solution (TMB as substrate) was added to each well for color development; subsequently, 2M H₂SO₄ stop solution was added to each well to stop the reaction; the OD value of each well was measured at a wavelength of 450 nm immediately with a microplate reader (Molecular Device); the data were analyzed to detemine positive clones.

The positive clones selected were not necessarily monoclonal, and some clones may be unstable, so subcloning needs to be carried out in time to obtain a monoclonal antibody finally. The subcloning was carried out by the limiting dilution method, with the process as follows: hybridoma cell suspension of the positive clones was prepared, and the cell density was adjusted to a dilution of 10 or 20 cells per milliliter with HT medium (with the preparation method of HT medium as follows: 100×HT solution was diluted to the required concentration with RPMI-1640 complete medium, and 100×HT was purchased from Thermo Fisher Scientific); each dilution was inoculated into a 96-well plate, with 0.2 ml per well and an average of 2 or 4 hybridoma cells per well; placed in a CO₂ cell incubator for 7-10 days; when there were clones visible to the naked eye, the cells were observed under an inverted microscope, and the wells where only single clone grew were marked, and the supernatant was taken for ELISA detection (with the detection method as described above); the positive wells detected by ELISA were amplified and cultured in 24-well plates, and a new round of subcloning was performed by the limiting dilution method; no less than 24 wells were selected for ELISA detection. When the detection shows 100% positive, it indicates that a monoclonal hybridoma cell line stably expressing the target antibody has been obtained. The monoclonal hybridoma cell line was cultured and expanded, and cryopreserved.

The stable monoclonal cell line was cultured and expanded in a serum-free medium HybriGRO SF (purchased from Coming) for about 5-7 days. The cell culture supernatant was centrifuged and filtered to remove the precipitate, and then Protein G (purchased from GE Healthcare) was used to purify the murine antibody. After several fusions and screenings, the present invention successfully established more than 200 stable hybridoma monoclonal cell lines, and purified and obtained corresponding murine antibodies for further experiments.

### Example 3. Effect of murine anti-human LAG-3 antibody on mixed lymphocyte reaction

Herein, the activity of mouse anti-human LAG-3 antibody was evaluated using mixed lymphocyte reaction (MLR), and Anti-LAG3.5 was added as a positive control.

The MLR experimental method is described as follows: Peripheral blood mononuclear cells (PBMC) were isolated from human blood with Histopaque (purchased from Sigma), and the monocyte subpopulations in PBMC were seperated by the adherence method, then IL-4 and GM-CSF were combined to induce the monocytes to differentiate into induced dendritic cells (p38 MAPK-inhibited dendritic cells induce superior antitumour immune responses and overcome regulatory T-cell-mediated immunosuppression. Nat Commun. 2014 Jun 24; 5:4229. doi: 10.1038/ncomms5229.); seven days later, the above induced dendritic cells were digested and collected; PBMCs were isolated from the blood of other donors using the above method, and then CD4 positive T cells were isolated from them with MACS magnets and CD4 MicroBeads (purchased from Miltenyi Biotec); the induced dendritic cells (10⁴ cells/well) and the isolated CD4 positive T cells (10⁵ cells/well) were mixed at a ratio of 10:1 and then inoculated into 96-well plates with 150 µl per well; after 2 hours, 50 µl of murine anti-LAG-3 antibody diluted with AIM-V medium was added to the above 96-well plates; at the same time, the positive control antibody Anti-LAG3.5 and an irrelevant isotype control antibody that does not bind to LAG-3 were provided; the 96-well plates were incubated in a cell incubator (37°C, 5% CO₂) for 3-4 days; an appropriate amount of culture supernatant was taken and used to detect the secretion of IL-2 by double-antibody sandwich ELISA (related detection reagents purchased from BD Biosciences); a multifunctional microplate reader (Molecular Device) was used to read OD450, and GraphPad Prism6 was used for data analysis and graphing. In this example, serum-free medium AIM-V (purchased from Thermo Fisher Scientific) was used for cell culture.

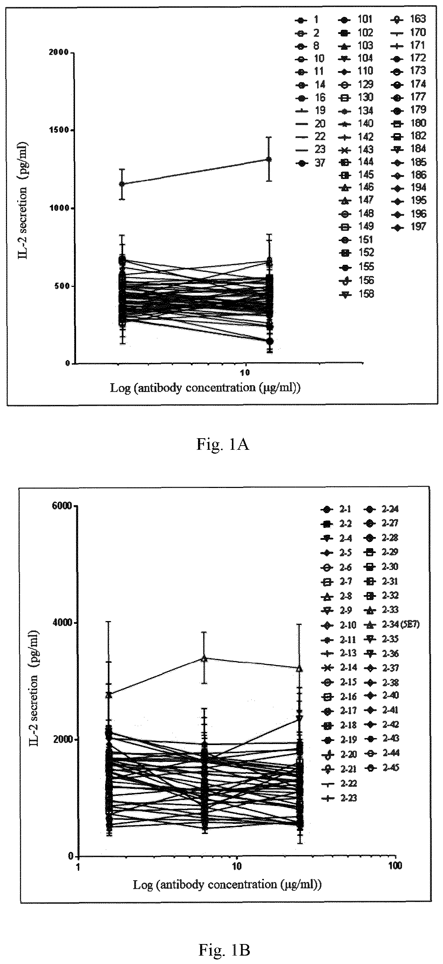

The experimental results are shown in Figs. 1A and 1B. By two rounds of screening (Figs. 1A and 1B), two murine anti-human LAG-3 antibodies that significantly enhance the secretion of IL-2 by MLR were obtained, namely monoclonal antibodies No. 134 and No. 2-34 (i.e., 5E7).

### Example 4. Determination of nucleotide sequence and amino acid sequence of the preferred murine anti-human LAG-3 antibody

According to the screening results of Example 3, the monoclonal antibodies No. 134 and No. 5E7 were finally selected as lead antibodies. Total RNAs were extracted from the hybridoma monoclonal cell lines corresponding to the monoclonal antibodies No. 134 and No. 5E7 using TRIzol (purchased from Thermo Fisher Scientific), and mRNAs were reverse transcribed into cDNAs using a reverse transcription kit (purchased from Takara). By the primer combination reported in the literature (Antibody Engineering, Volume 1, Edited by Roland Kontermann and Stefan Dübel; the sequences of the primer combination from page 323), the genes of light chain variable region and heavy chain variable region of the murine anti-human LAG-3 antibody were amplified by PCR, and then the PCR products were cloned into the pMD18-T vector, and the variable region genes were sequenced and analyzed.

The sequence information of Clone No. 134 is as follows: the heavy chain variable region gene sequence is 360 bp in length, encoding 120 amino acid residues, the nucleotide sequence is shown in SEQ ID NO: 1, and the amino acid sequence is shown in SEQ ID NO: 2; the light chain variable region gene sequence is 321 bp in length, encoding 107 amino acid residues, the nucleotide sequence is shown in SEQ ID NO: 3, and the amino acid sequence is shown in SEQ ID NO: 4.
Amino acid sequence of heavy chain variable region of Clone No. 134 
EVQLQQSGPVLVKPGASVKMSCKASGYTLTAYYMNWVKQSRGKSLEWIGVINPYNGDSSY
NQKFKGKATLTVDKSSSTAYMELNSLTSEDSAVYYCARDDGYYRWYFDVWGTGTTVTVSS
(SEQ ID NO: 2)
Amino acid sequence of light chain variable region of Clone No. 134

DIQMTQSPSSLSASLGERVSLTCRASQDIGSRLNWLQQGPDGSIKRLIYATSSLESGVPKRFS
GSRSGSDYFLTISSLESEDFVDYYCLQCGSSPPTFGGGTKLEIK (SEQ ID NO: 4)

The sequence information of Clone No. 5E7 is as follows: the heavy chain variable region gene sequence is 348 bp in length, encoding 116 amino acid residues, the nucleotide sequence is shown in SEQ ID NO: 5, and the amino acid sequence is shown in SEQ ID NO: 6; the light chain variable region gene sequence is 321 bp in length, encoding 107 amino acid residues, the nucleotide sequence is shown in SEQ ID NO: 7, and the amino acid sequence is shown in SEQ ID NO: 8.
Amino acid sequence of heavy chain variable region of Clone No. 5E7 
EVKLVESGGGLVKPGGSLKLSCAASGFTFSDDYMAWFRQTPEKRLEWVASISHGGDYIYYA
DNLKGRFTISRDNAKNTLYLQMSSLKSEDTAIYFCSRDRRSIDYWGQGTTLTVSS (SEQ ID
NO: 6)
Amino acid sequence of light chain variable region of Clone No. 5E7

DIQMTQITSSLSASLGDRVTITCRASQDISNYLSWYQQKPDGTIKLLIYYTSRLHSGVPSRFS
GSGSGTDYSLTISDLEQEDIATYFCQQGKTLPYTFGGGTKLERK (SEQ ID NO: 8)

### Example 5. Humanization of murine anti-human LAG-3 antibody

The amino acid sequences of the light chain variable region and the heavy chain variable region in Example 4 were analyzed, and three complementarity-determining regions (CDRs) and four frame regions (FRs) of the antibodies No. 134 and 5E7 were determined according to the Kabat numbering.

Wherein, the amino acid sequences of the heavy chain complementarity-determining regions of Clone No. 134 are: HCDR1: AYYMN (SEQ ID NO: 9), HCDR2: VINPYNGDSSYNQKFKG (SEQ ID NO: 10) and HCDR3: DDGYYRWYFDV (SEQ ID NO: 11), the amino acid sequences of the light chain complementarity-determining regions are: LCDR1: RASQDIGSRLN (SEQ ID NO: 12), LCDR2: ATSSLES (SEQ ID NO: 13) and LCDR3: LQCGSSPPT (SEQ ID NO: 14).

Wherein, the amino acid sequences of the heavy chain complementarity-determining regions of Clone No. 5E7 are: HCDR1: DDYMA (SEQ ID NO: 15), HCDR2: SISHGGDYIYYADNLKG (SEQ ID NO: 16) and HCDR3: DRRSIDY (SEQ ID NO: 17), the amino acid sequences of the light chain complementarity-determining regions are: LCDR1: RASQDISNYLS (SEQ ID NO: 18), LCDR2: YTSRLHS (SEQ ID NO: 19) and LCDR3: QQGKTLPYT (SEQ ID NO: 20).

The homology comparison of the amino acid sequence of the heavy chain variable region of the antibody No. 134 with human immunoglobulin variable region germline sequence (Germline) was performed on https://www.ncbi.nlm.nih.gov/igblast/. IGHV1-46*01, which has the highest homology with the antibody No. 134, was selected as a humanized template of the heavy chain variable region, using the CDRs of the heavy chain of the murine antibody No. 134 to replace the corresponding CDRs in IGHV1-46*01 to construct a heavy chain CDR-grafted variable region. Similarly, by homology comparison, IGKV1-39*01 germline sequence was selected as a humanized template of the light chain variable region, using the CDRs of the light chain of the murine antibody No. 134 to replace the corresponding CDRs in IGKV1-39*01 to construct a light chain CDR-grafted variable region. Then, on the basis of the CDR-grafted variable regions of the heavy chain and light chain, some amino acid sites in the framework regions were subjected to back mutation. Back mutation refers to mutation of certain amino acids (important amino acids for maintaining antibody structure and affinity) in the CDR-grafted variable framework region to the amino acids at the corresponding position in the murine framework region. When back mutation is performed, the amino acid sequence is numbered by Kabat rule and the position of each amino acid is indicated by Kabat numbering. Preferably, for the heavy chain CDR-grafted variable region, G at position 44 was back mutated to S, M at position 48 was back mutated to I, V at position 67 was back mutated to A, M at position 69 was back mutated to L, R at position 71 was back mutated to V, T at position 73 was back mutated to K, V at position 78 was back mutated to A. Preferably, for the light chain CDR-grafted variable region, Y at position 36 was back mutated to L, A at position 43 was back mutated to S, P at position 44 was back mutated to I, L at position 46 was back mutated to R, G at position 66 was back mutated to R, T at position 69 was back mutated to S, F at position 71 was back mutated to Y. It should be noted that since the CDR3 (SEQ ID NO: 14) of the light chain of the antibody No. 134 contains an extra cysteine (at position 91 by Kabat numbering, C91), which may cause abnormal structure of the antibody, herein, it was mutated to serine (C91S, SEQ ID NO: 43). The heavy chain and light chain CDR-grafted variable regions carrying the above-mentioned back mutations were added with the amino acid sequence at the end, respectively: WGQGTKVEIK (SEQ ID NO: 44) and FGQGTKVEIK (SEQ ID NO: 45), to construct complete humanized heavy chain and light chain variable regions. Relevant gene sequences were subjected to codon optimization and gene synthesis by Sangon Biotech (Shanghai) Co., Ltd..

The gene sequence of the humanized heavy chain variable region (134-Hu-VH) of the resulting antibody No.134 is 354 bp in length encoding 118 amino acid residues, the nucleotide sequence is shown in SEQ ID NO: 21, and the amino acid sequence is shown in SEQ ID NO: 22; the gene sequence of the humanized light chain variable region (134-Hu-VL-C91S) of the antibody No.134 is 321 bp in length, encoding 107 amino acid residues, and the nucleotide sequence is shown in SEQ ID NO: 23, and the amino acid sequence is shown in SEQ ID NO:24.
Amino acid sequence of humanized heavy chain variable region of 134 (134-Hu-VH) QVQLVQSGAEVKKPGASVKVSCKASGYTLTAYYMNWVRQAPGQSLEWIGVINPYNGDSS YNOKFKGRATLTVDKSTSTAYMELSSLRSEDTAVYYCARDDGYYRWYFDVWGOGTLVTV SS (SEQ ID NO: 22, where the underlined part is heavy chain complementarity-determining region)
Amino acid sequence of humanized light chain variable region of 134 (134-Hu-VL-C91S) DIQMTQSPSSLSASVGDRVTITCRASQDIGSRLNWLQQKPGKSIKRLIYATSSLESGVPSRFS GSRSGSDYTLTISSLQPEDFATYYCLQSGSSPPTFGQGTKVEIK (SEQ ID NO: 24, where the underlined part is the light chain complementarity-determining region)

The antibody No. 5E7 was humanized in the same way. IGHV3-21*01, which has the highest homology with the antibody No. 5E7, was selected as a humanized template of the heavy chain variable region, using the CDRs of the heavy chain of the murine antibody No. 5E7 to replace the corresponding CDRs in IGHV3-21*01, to construct a heavy chain CDR-grafted variable region. Similarly, by homology comparison, IGKV1-33*01 germline sequence was selected as a humanized template of the light chain variable region, using the CDRs of the light chain of the murine antibody No. 5E7 to replace the corresponding CDRs in IGKV1-33*01, to construct a light chain CDR-grafted variable region. Then, on the basis of the CDR-grafted variable regions of the heavy chain and light chain, some amino acid sites in the framework regions were subjected to back mutation. When back mutation was performed, the amino acid sequence was numbered by Kabat rule and the position of each amino acid was indicated by Kabat numbering. Preferably, for the heavy chain CDR-grafted variable region, V at position 37 was back mutated to F, G at position 44 was back mutated to R, S at position 49 was back mutated to A, Y at position 91 was back mutated to F, A at position 93 was back mutated to S. Preferably, for the light chain CDR-grafted variable region, A at position 43 was back mutated to T, P at position 44 was back mutated to I, F at position 71 was back mutated to Y, Y at position 87 was back mutated to F. The heavy chain and light chain CDR-grafted variable regions carrying the above-mentioned back mutations were added with the amino acid sequence at the end, respectively: WGQGTKVEIK (SEQ ID NO: 44) and FGQGTKVEIK (SEQ ID NO: 45) to construct complete humanized heavy chain and light chain variable regions. Relevant gene sequences were subjected to codon optimization and gene synthesis by Sangon Biotech (Shanghai) Co., Ltd..

The gene sequence of the humanized heavy chain variable region (5E7-Hu-VH) of the resulting antibody No. 5E7 is 354 bp in length, encoding 118 amino acid residues, the nucleotide sequence is shown in SEQ ID NO: 25, and the amino acid sequence is shown in SEQ ID NO: 26; the humanized light chain variable region (5E7-Hu-VL) gene sequence of the antibody No. 5E7 is 321 bp in length, encoding 107 amino acid residues, and the nucleotide sequence is shown in SEQ ID NO: 27, and the amino acid sequence is shown in SEQ ID NO:28.
Amino acid sequence of humanized heavy chain variable region of 5E7 (5E7-Hu-VH) EVOLVESGGGLVKPGGSLRLSCAASGFTFSDDYMAWFRQAPGKRLEWVASISHGGDYIYY ADNLKGRFTISRDNAKNSLYLQMNSLRAEDTAVYFCSRDRRSIDYWGQGTLUTVSS (SEQ ID NO: 26, where the underlined part is the heavy chain complementarity-determining region)
Amino acid sequence of humanized light chain variable region of 5E7 (5E7-Hu-VL) DIQMTQSPSSLSASVGDRVTITCRASQDISNYLSWYQQKPGKTIKLLIYYTSRLHSGVPSRFS GSGSGTDYTFTISSLQPEDIATYFCQQGKTLPYTFGQGTKVEIK (SEQ ID NO: 28, where the underlined part is the light chain complementarity-determining region)

The above synthesized humanized heavy chain variable region of the antibody No.134 was linked to human immunoglobulin IgG4 constant region (the nucleotide sequence is shown in SEQ ID NO: 29, and the amino acid sequence is shown in SEQ ID NO: 30), to construct a complete humanized heavy chain of the antibody No. 134, named 134-Hu-IgG4-HC (the nucleotide sequence is shown in SEQ ID NO: 31, and the amino acid sequence is shown in SEQ ID NO: 32); the humanized light chain variable region of the antibody No.134 was linked to human immunoglobulin Kappa chain constant region (the nucleotide sequence is shown in SEQ ID NO: 33, and the amino acid sequence is shown in SEQ ID NO: 34), to construct a complete humanized light chain of the antibody No. 134, named 134-Hu-LC-C91S (the nucleotide sequence is shown in SEQ ID NO: 35, and the amino acid sequence is shown in SEQ ID NO: 36).
Amino acid sequence of constant region of human immunoglobulin IgG4 
ASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPPCPAPEFLGGPSVFLFPPK
PKDTLMISRTPEVTCVVVDVSQEDPEVQFNWYVDGVEVHNAKTKPREEQFNSTYRVVSVL
TVLHQDWLNGKEYKCKVSNKGLPSSIEKTISKAKGQPREPQVYTLPPSQEEMTKNQVSLTC
LVKGFYPSDIAVEWESNGQPENNYKTTPPVLDSDGSFFLYSRLTVDKSRWQEGNVFSCSVM
HEALHNHYTQKSLSLSLGK (SEQ ID NO: 30)
Amino acid sequence of humanized heavy chain 134-Hu-IgG4-HC of 134 QVQLVQSGAEVKKPGASVKVSCKASGYTLTAYYMNWVRQAPGQSLEWIGVINPYNGDSS YNQKFKGRATLTVDKSTSTAYMELSSLRSEDTAVYYCARDDGYYRWYFDVWGQGTLVTV SSASTKGPSVFPLAPCSRSTSESTAALGCLVKDYFPEPVTVSWNSGALTSGVHTFPAVLQSSG LYSLSSVVTVPSSSLGTKTYTCNVDHKPSNTKVDKRVESKYGPPCPPCPAPEFLGGPSVFLFP PKPKDTLMISRTPEVTCWVDVSQEDPEVQFNWYVDGVEVHNAKTKPREEQFNSTYRVVS VLTVLHQDWLNGKEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDKSRWQEGNVFSCSV MHEALHNHYTQKSLSLSLGK (SEQ ID NO: 32, where the underlined part is the heavy chain complementarity-determining region)
Amino acid sequence of constant region of human immunoglobulin Kappa chain 

 (SEQ ID NO: 34)
Amino acid sequence of humanized light chain 134-Hu-LC-C91S of 134 DIQMTQSPSSLSASVGDRVTITCRASQDIGSRLNWLQQKPGKSIKRLIYATSSLESGVPSRFS GSRSGSDYTLTISSLQPEDFATYYCLQSGSSPPTFGQGTKVEIKRTVAAPSVFIFPPSDEQLKS GTASVVCLLNNFYPREAKVQWKVDNALQSGNSQESVTEQDSKDSTYSLSSTLTLSKADYE KHKVYACEVTHQGLSSPVTKSFNRGEC (SEQ ID NO: 36, where the underlined part is the light chain complementarity-determining region)

The above synthesized humanized heavy chain variable region of the antibody No. 5E7 was linked to human immunoglobulin IgG4 constant region (the nucleotide sequence is shown in SEQ ID NO: 29, and the amino acid sequence is shown in SEQ ID NO: 30), to construct a complete humanized heavy chain of the antibody No. 5E7, named 5E7-Hu-IgG4-HC (the nucleotide sequence is shown in SEQ ID NO: 37, and the amino acid sequence is shown in SEQ ID NO: 38); the humanized light chain variable region of the antibody No. 5E7 was linked to human immunoglobulin Kappa chain constant region (the nucleotide sequence is shown in SEQ ID NO: 33, and the amino acid sequence is shown in SEQ ID NO: 34), to construct a complete humanized light chain of the antibody No. 5E7, named 5E7-Hu-LC (the nucleotide sequence is shown in SEQ ID NO: 39, and the amino acid sequence is shown in SEQ ID NO: 40).
Amino acid sequence of humanized heavy chain 5E7-Hu-IgG4-HC of 5E7 EVQLVESGGGLVKPGGSLRLSCAASGFTFSDDYMAWFRQAPGKRLEWVASISHGGDYIYY ADNLKGRFTISRDNAKNSLYLQMNSLRAEDTAVYFCSRDRRSIDYWGQGTLVTVSSASTKG PSVFPLAPCSRSTSESTAALGCLVKDYFPEPVTVSWNSGALTSGVHTFPAVLQSSGLYSLSSV VTVPSSSLGTKTYTCNVDHKPSNTKVDKRVESKYGPPCPPCPAPEFLGGPSVFLFPPKPKDT LMISRTPEVTCVVVDVSQEDPEVQFNWYVDGVEVHNAKTKPREEQFNSTYRVVSVLTVLH QDWLNGKEYKCKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGNVFSCSVMHEAL HNHYTQKSLSLSLGK (SEQ ID NO: 38, where the underlined part is the heavy chain complementarity-determining region)
Amino acid sequence of humanized light chain 5E7-Hu-LC of 5E7 DIQMTQSPSSLSASVGDRVTITCRASQDISNYLSWYQQKPGKTIKLLIYYTSRLHSGVPSRFS GSGSGTDYTFTISSLQPEDIATYFCQQGKTLPYTFGQGTKVEIKRTVAAPSVFIFPPSDEQLKS GTASVVCLLNNFYPREAKVQWKVDNALQSGNSQESVTEQDSKDSTYSLSSTLTLSKADYE KHKVYACEVTHQGLSSPVTKSFNRGEC (SEQ ID NO: 40, the underlined part is the light chain complementarity-determining region)

The above-mentioned 134-Hu-IgG4-HC and 134-Hu-LC-C91S genes were constructed into the pTT5 expression vector and the constructed expression vectors were co-transfected into HEK293E cells with PEI for transient expression, respectively. HEK293E cells were cultured in a serum-free medium, FreeStyle 293 Expression Medium (purchased from Thermo Fisher Scientific). After 5 days, the cells were centrifuged and the cell supernatant was collected. The antibody was purified by Protein A affinity chromatography, and the resulting antibody was named 134-Hu-IgG4-C91S. The same method was used to construct the expression vectors of 5E7 humanized heavy chain and light chain, and express and purify the antibody, and the resulting antibody was named 5E7-Hu-IgG4.

In addition, the heavy chain variable region and the light chain variable region of the murine antibody No. 134 were spliced with the heavy chain constant region of the human immunoglobulin IgG4 (the nucleotide sequence is shown in SEQ ID NO: 29, and the amino acid sequence is shown in SEQ ID NO: 30) and the Kappa light chain constant region (the nucleotide sequence is shown in SEQ ID NO: 33, and the amino acid sequence is shown in SEQ ID NO: 34) by recombinant PCR, and constructed into the pTT5 expression vector, respectively. The antibody was expressed and purified by the method described above, and the resulting chimeric antibody was named 134-Chimeric. The chimeric antibody 5E7-Chimeric of the antibody No. 5E7 was obtained by the same method.

The effect of humanization on the affinity between antibody and antigen was detected by ELISA. The microplates were coated with LAG-3-ECD-His (LAG-3 extracellular segment with His tag, purchased from Sino Biological Inc.) (10 ng/well), incubated at room temperature for 2 hours, and then washed with PBST (containing 0.05% Tween-20 in PBS); 200 µl of blocking solution (PBST solution containing 1% BSA) was added to each well for blocking; the plates were washed with PBST and pat dried; Anti-human LAG-3 antibody serially diluted with the blocking solution was added to the microplates, incubated at room temperature for about 1 hour, and then the microplates were washed; Goat-Anti-Human IgG (Fc specific) secondary antibody (purchased from Sigma) diluted with the blocking solution was added, incubated at room temperature for about 1 hour, and then the microplates were washed; 100 µl of chromogenic solution (TMB substrate) was added to each well for color development; then 50 µl of 2M H₂SO₄ was added to each well to stop the color reaction; SpectraMax 190 (Molecular Device) was used to measure the OD value of each well at a wavelength of 450 nm; GraphPad Prism6 was used to analyze data and graph, and calculate EC50.

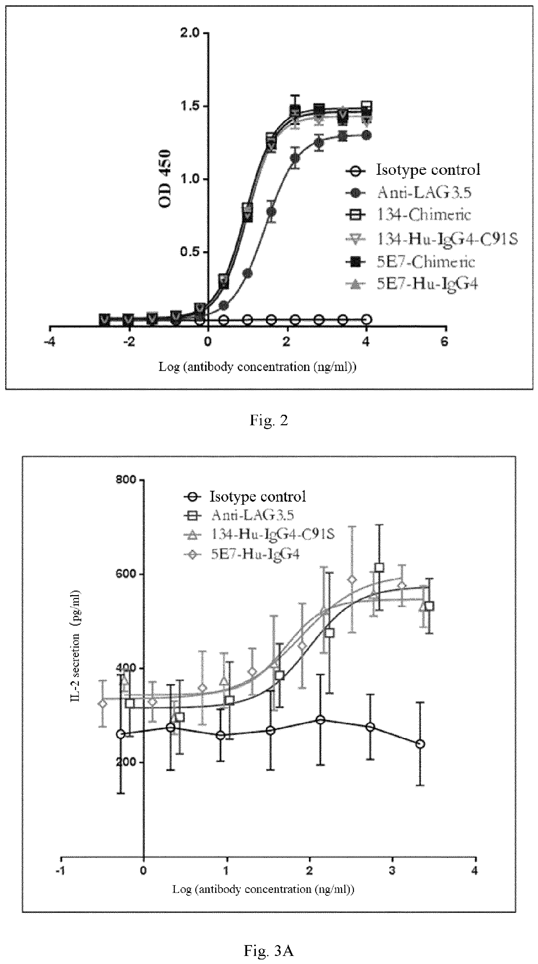

The experimental results are shown in Fig. 2. Anti-LAG3.5, 134-Chimeric, 134-Hu-IgG4-C91S, 5E7-Chimeric and 5E7-Hu-IgG4 can effectively bind to LAG-3, with an EC₅₀ of 27.63 ng/ml, 8.685 ng/ml, 8.782 ng/ml, 9.741 ng/ml and 9.078 ng/ml, respectively. The EC50 of the corresponding humanized antibody was basically the same as that of the chimeric antibody, indicating that the humanized modification does not reduce the affinity between antibody and antigen. Among them, the isotype control is an IgG4 antibody that does not bind to LAG-3.

### Example 6. Examination of the enhancing effect of humanized anti-human LAG-3 antibody on IL-2 secretion by PBMC

Staphylococcal Enterotoxin A (SEA) is a superantigen that can cross-link the MHC class II molecules of antigen presenting cells (APC) and the T cell receptors (TCR) on the surface of T cells, and thus activate a large number of T cell clones and strongly stimulate the immune response. Activated T cells express LAG-3, and LAG-3 can bind to the MHC class II molecules on the surface of APC, and thus exerts an immunosuppressive effect. In this example, after stimulating PBMC with SEA, anti-human LAG-3 antibody was added. The functional activity of the humanized anti-human LAG-3 antibody was evaluated by detecting the secretion of IL-2.

The experimental method is described as follows: SEA gene with a histidine tag was cloned into the pET28a vector, the protein was expressed with E. coli system, and then SEA was purified with a nickel affinity chromatography column; PBMC was isolated from human blood with Histopaque, washed with PBS and centrifuged; the cells were resuspended in RPMI-1640 medium (containing 10% fetal bovine serum), the SEA prepared above (SEQ ID NO: 52) was added to a final concentration of 1 ng/ml, and then PBMC was inoculated into round-bottom 96-well plates at 2×10⁵ cells per well, 150 µl per well; then 50 µl of serially diluted anti-human LAG-3 antibody was added to each well, and the 96-well plates were incubated in a incubator (37 °C, 5% CO₂) for 2 days; appropriate amount of culture supernatant was taken for the detection of IL-2 secretion by double antibody sandwich ELISA (related detection reagents were purchased from BD Biosciences). Microplate reader (Molecular Device) was used to read OD450, and GraphPad Prism6 was used for data analysis and graphing.

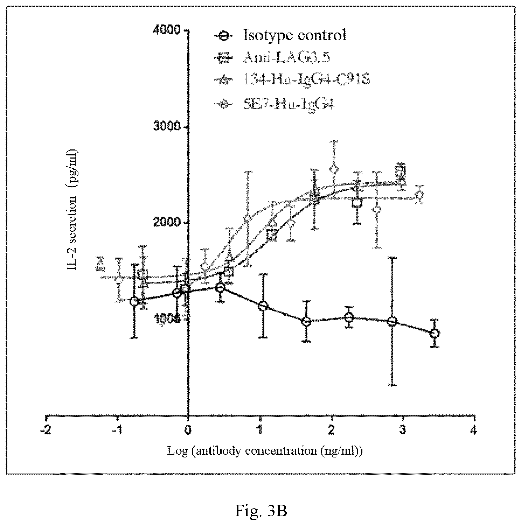

The PBMCs in Figs. 3A and 3B were from different donors. Experimental results show that Anti-LAG3.5, 134-Hu-IgG4-C91S and 5E7-Hu-IgG4 effectively enhanced the secretion of IL-2 by SEA-stimulated PBMC. In the experiment shown in Fig. 3A, the EC50 of Anti-LAG3.5, 134-Hu-IgG4-C91S and 5E7-Hu-IgG4 were 98.09 ng/ml, 50.71 ng/ml and 78.12 ng/ml, respectively; in the experiment shown in Fig. 3B, the EC50 of the above three antibodies were 16.21 ng/ml, 10.85 ng/ml and 3.068 ng/ml, respectively. The results of both experiments show that the functional activity of 134-Hu-IgG4-C91S and 5E7-Hu-IgG4 on stimulating activated PBMC to secrete IL-2 is stronger than that of Anti-LAG3.5. Among them, the isotype control is an IgG4 antibody that does not bind to LAG-3.

### Example 7. Binding of humanized anti-human LAG-3 antibody to LAG-3 on the cell surface

Superantigen SEA can activate T cell clones in PBMC, and the activated T cells will express LAG-3. In this example, flow cytometry was used to determine the binding of the humanized anti-human LAG-3 antibody of the present invention to LAG-3 on the cell surface.

The experimental procedures are as follows: PBMCs were isolated from human blood with Histopaque, washed twice with PBS, and centrifuged to collect the cells; the cells were resuspended in RPMI-1640 medium (containing 10% fetal bovine serum), and SEA was added to a final concentration of 1ng/ ml, then PBMC was inoculated into round-bottomed 96-well plates with 2×10⁵ cells per well; the 96-well plates were incubated in a incubator (37°C, 5% CO₂) for 2 days; the cells in the 96-well plates were washed with PBS, and centrifuged, and the supernatant was discarded; the antibody was biotinylated with Biotin N-hydroxysuccinimide ester (Cat. No./Specification: H1759-100MG, purchased from Sigma) according to the instructions provided by the manufacturer; serially diluted anti-human LAG-3 antibody labeled with biotin was added and incubated for about 1 hour, then the cells were washed twice with PBS; PE-Streptavidin (purchased from BD Biosciences) appropriately diluted with PBS containing 1% BSA was added to the above cells and incubated for about 1 hour, then the cells were washed twice with PBS; 4% paraformaldehyde was added to fix the cells. Flow cytometer (CytoFLEX Cytometer System, purchased from Beckman Coulter) was used to measure the PE fluorescence intensity of the cells; Flow cytometer analysis software was used to process the experimental data and calculate the mean fluorescence intensity; GraphPad Prism6 was used to analyze data and graph, and calculate EC50.

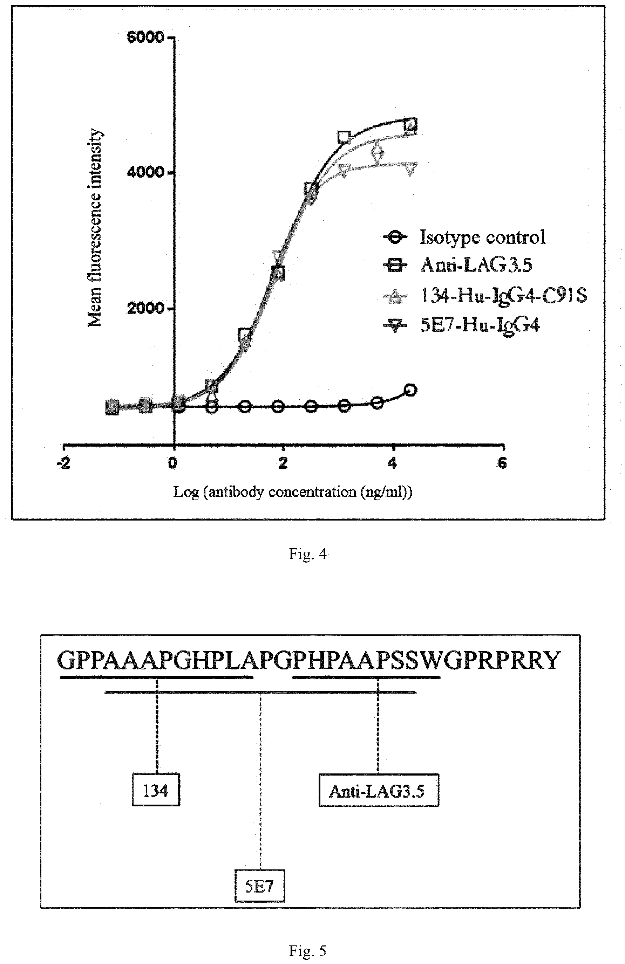

The experimental results are shown in Fig. 4. Anti-LAG3.5, 134-Hu-IgG4-C91S and 5E7-Hu-IgG4 can effectively bind to LAG-3 on the cell surface, with an EC50 of 71.68 ng/ml and 80.45 ng/ml and 53.13 ng/ml, respectively. The binding abilities of the three are basically the same. Among them, the isotype control is an IgG4 antibody that does not bind to LAG-3, and the ordinate is the mean fluorescence intensity.

### Example 8. Epitope analysis of anti-human LAG-3 antibody

The first immunoglobulin-like domain of the extracellular segment of LAG-3 contains an exposed "extra loop", having the amino acid sequence of: GPPAAAPGHPLAPGPHPAAPSSWGPRPRRY (SEQ ID NO: 46). According to reports in the literature, this peptide plays an important role in the binding of LAG-3 to MHC-II molecules (Baixeras E, Huard B, Miossec C, et al. Characterization of the lymphocyte activation gene 3-encoded protein. A new ligand for human leukocyte antigen class II antigens.[J]. Journal of Experimental Medicine, 1992, 176(2): 327-337.). In order to test the binding of the anti-human LAG-3 antibody of the present invention to the peptide and determine the binding epitope of each antibody, the following peptide scanning experiment was carried out: a series of partially overlapping peptides were synthesized by chemical methods. These partially overlapping peptides covered the entire "extra loop" area, and a biotin tag was attached to the N-terminus of these peptides. Microplates were coated with Streptavidin (200 ng/well), washed, and then blocked with blocking solution (PBST solution containing 1% BSA); the biotinylated peptides were diluted to 1 µg/ml with the blocking solution and added to the microplates, incubated at room temperature for 1 hour to allow them to be captured by Streptavidin, and then the microplates were washed; the anti-human LAG-3 antibody serially diluted with the blocking solution was added, incubated at room temperature for about 1 hour, then the microplates were washed; Goat-Anti-Human IgG (Fc specific) secondary antibody (purchased from Sigma) appropriately diluted with the blocking solution was added to detect the binding of anti-human LAG-3 antibody to the peptides, incubated at room temperature for about 1 hour; 100 µl of chromogenic solution (TMB substrate) was added to each well for color development, and then 50 µl of 2 M H₂SO₄ stop solution was added to each well to terminate the reaction. SpectraMax 190 (Molecular Devices, Inc.) was used to measure the OD value of each well at a wavelength of 450 nm; GraphPad Prism6 was used to analyze data and graph, and calculate EC50. The results of the peptide scanning experiment are summarized in Table 1.

**Table 1. Summary of the results of peptide scanning experiment**

| **Polypeptide amino acid sequence** | | | | | | | | | | | | | **Anti-LAG3-5** | **134-Hu-IgG4-C91S** | **5E7-Hu-IgG4** |
|---|---|---|---|---|---|---|---|---|---|---|---|---|---|---|---|
| GPPAAAPGHPLAPGPHPAAPSSWGPRPRRY | | | | | | | | | | | | | +++ | +++ | +++ |
| GPPAAAPGHPLA | | | | | | | | | | | | | - | +++ | - |
| | PAAAPGHPLAPG | | | | | | | | | | | | - | ++ | - |
| | | | AAPGHPLAPGPH | | | | | | | | | | - | + | - |
| | | | | | PGHPLAPGPHPA | | | | | | | | - | - | - |
| | | | | | | | HPLAPGPHPAAP | | | | | | - | - | + |
| | | | | | | | | LAPGPHPAAPSS | | | | | - | - | + |
| | | | | | | | | | PGPHPAAPSSWG | | | | +++ | - | - |
| | | | | | | | | | | PHPAAPSSWGPR | | | +++ | - | - |
| | | | | | | | | | | | PAAPSSWGPRPR | | - | - | - |
| | | | | | | | | | | | | APSSWGPRPRRY | - | - | - |
| | | AAAPGHPLAPGPHPAAPSS | | | | | | | | | | | - | - | +++ |
| | | | AAPGHPLAPGPHPAAPSSW | | | | | | | | | | +++ | - | ++ |
| | | | | APGHPLAPGPHPAAPSSWG | | | | | | | | | +++ | - | + |
| | | | | | PGHPLAPGPHPAAPSSWGP | | | | | | | | +++ | - | + |
| | | | | | | GHPLAPGPHPAAPSSWGPR | | | | | | | +++ | - | + |
| | | | | | | | HPLAPGPHPAAPSSWGPRP | | | | | | +++ | - | + |

| | | | | | | | | | | | | | | | |
|---|---|---|---|---|---|---|---|---|---|---|---|---|---|---|---|
| Note: "-" means no obvious binding, "+" means obvious binding, more "+" means stronger binding; the smaller the EC50, the more "+". | | | | | | | | | | | | | | | |

If the antibody obviously binds to several peptides, then the shortest overlapping peptide is the epitope of the antibody. The experimental results of Table 1 show that the epitope of Anti-LAG3.5 antibody is PHPAAPSSW (SEQ ID NO: 47), the epitope of the antibody No. 134 is GPPAAAPGHPLA (SEQ ID NO: 48); and the epitope of the antibody No. 5E7 is AAAPGHPLAPGPHPAAPSS (SEQ ID NO: 49). The related results are shown in Fig. 5.

### Example 9. Blocking effect of humanized anti-human LAG-3 antibody on the binding of LAG-3 to Raji cells

Raji cells are a type of Burkitt lymphoma cells that originate from B lymphocytes and highly express MHC-II molecules. In this example, flow cytometry was used to determine the blocking effect of the humanized anti-human LAG-3 antibody of the present invention on the binding of LAG-3 to MHC-II molecules on the surface of Raji cells.

The experimental procedures are as follows: Raji cells (purchased from American Type Culture Collection, abbreviated as ATCC; ATCC^{®} CCL-86^{™}) were washed twice with PBS, and then inoculated into round-bottomed 96-well plates with 2×10⁵ cells per well, centrifuged, and then the supernatant was discarded; the biotinylated LAG-3 (purchased from ACROBiosystems) was diluted to 0.5 µg/ml with PBS containing 1% BSA, and then the anti-human LAG-3 antibody was serially diluted in this solution; then the prepared mixture containing biotinylated LAG-3 and anti-human LAG-3 antibody was added to the above cells, incubated at room temperature for about 1 hour, then the cells were washed twice with PBS; PE-Streptavidin (purchased from BD Biosciences) was appropriately diluted with PBS containing 1% BSA and added to the above cells, incubated for about 1 hour, the cells were washed twice with PBS; 4% paraformaldehyde was added to fix the cells. Flow cytometer (CytoFLEX Cytometer System, purchased from Beckman Coulter) was used to measure the PE fluorescence intensity of the cells; Flow cytometer analysis software was used to process the experimental data and calculate the mean fluorescence intensity; GraphPad Prism6 was used to analyze data and graph, and calculate EC50.

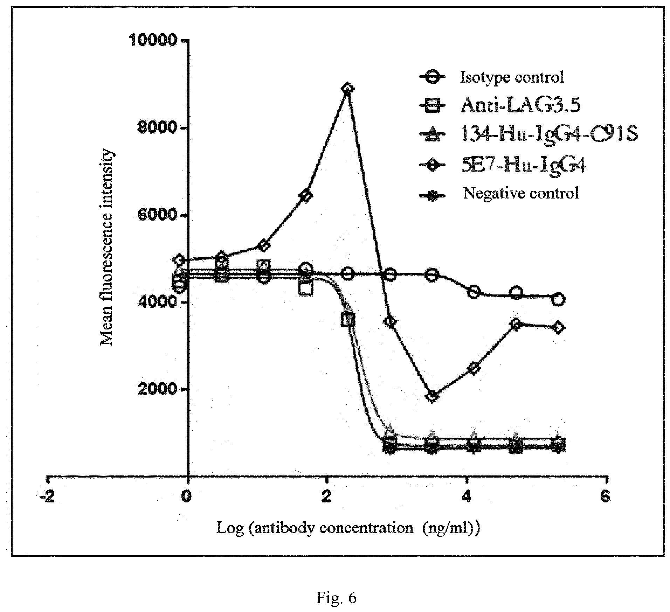

The experimental results are shown in Fig. 6. Both Anti-LAG3.5 and 134-Hu-IgG4-C91S can effectively block the binding of LAG-3 to Raji cells, with IC50s of 256.9 ng/ml and 288.8 ng/ml, respectively. The blocking abilities of the both are basically the same. The effect of 5E7-Hu-IgG4 on the binding of LAG-3 to Raji cells is different from that of Anti-LAG3.5 and 134-Hu-IgG4-C91S: at high concentrations, 5E7-Hu-IgG4 can only partially block the binding of LAG-3 to Raji Cells; at low concentrations, 5E7-Hu-IgG4 can instead promote the binding of LAG-3 to Raji cells. And, the isotype control is an IgG4 antibody that does not bind to human LAG-3, and the negative control represents the background fluorescence value of Raji cells in the absence of LAG-3.

### Example 10. Detection of the affinity by Biacore

In this example, the affinity of Anti-LAG3.5, 134-Hu-IgG4-C91S, and 5E7-Hu-IgG4 for LAG-3 was detected by the molecular interaction instrument Biacore 8K (GE healthcare).

Using HBS-EP+ pH7.4 as a dilution buffer, the anti-LAG-3 antibody sample was diluted to a concentration of 0.5 µg/mL, and the recombinant LAG-3 (purchased from SinoBiological, Cat. No.: 16498-H08H) was diluted to 1.024 nM, 2.56 nM, 6.4 nM, 16 nM, 40 nM and 100 nM, and an additional concentration was set as 0; 6M guanidine hydrochloride solution was used as regeneration buffer; on Biacore 8K, anti-LAG-3 antibody was captured by Protein A chip, and then recombinant LAG-3 was injected to obtain the binding-dissociation curve, the regeneration buffer was used for next cycle after the elution; Biacore 8K Insight Evaluation Software was used to analyze the data. The results are shown in Table 2

**Table 2. Determination of the affinity of anti-LAG-3 antibodies for human LAG-3**

| Sample Name | Kon (1/Ms) | Koff (1/s) | KD (M) |
|---|---|---|---|
| Anti-LAG3.5 | 6.67E+06 | 3.79E-03 | 5.68E-10 |
| 134-Hu-IgG4-C91S | 9.27E+06 | 4.43E-03 | 4.78E-10 |
| 5E7-Hu-IgG4 | 4.41E+06 | 1.84E-05 | 4.17E-12 |

The experimental results show that the equilibrium dissociation constants (KD) of Anti-LAG3.5, 134-Hu-IgG4-C91S and 5E7-Hu-IgG4 to LAG-3 were respectively 5.68E-10M, 4.78E-10M and 4.17E-12M, indicating that the affinity of 134-Hu-IgG4-C91S and 5E7-Hu-IgG4 for LAG-3 is higher than that of Anti-LAG3.5. 5E7-Hu-IgG4 has the smallest dissociation constant (Koff), since it has the smallest KD, its affinity is the highest.

### Example 11. Detection of the blocking effect of anti-human LAG-3 antibody on the binding of FGL1 to LAG-3 by flow cytometry

Recently, Chen Lieping's team discovered that FGL1 (Fibrinogen-like protein 1) is the main ligand of LAG-3 and clarified the basic principles of this tumor suppressor pathway (Reference: Wang J, Sanmamed MF, Datar I, et al. Fibrinogen-like protein 1 is a major immune inhibitory ligand of LAG-3[J]. Cell, 2019, 176(1-2): 334-347. e12.). In this example, the ability of Anti-LAG3.5, 134-Hu-IgG4-C91S and 5E7-Hu-IgG4 to block the interaction between FGL1 and LAG-3 was detected by flow cytometry.

The method of preparing recombinant human protein FGL1 is as follows: the human FGL1 sequence was obtained from http://www.uniprot.org (Entry: Q08830), having the amino acid sequence as follows: LEDCAQEQMRLRAQVRLLETRVKQQQVKIKQLLQENEVQFLDKGDENTVIDLGSKRQYAD CSEIFNDGYKLSGFYKIKPLQSPAEFSVYCDMSDGGGWTVIQRRSDGSENFNRGWKDYEN GFGNFVQKHGEYWLGNKNLHFLTTQEDYTLKIDLADFEKNSRYAQYKNFKVGDEKNFYEL NIGEYSGTAGDSLAGNFHPEVQWWASHQRMKFSTWDRDHDNYEGNCAEEDQSGWWFNR CHSANLNGVYYSGPYTAKTDNGIVWYTWHGWWYSLKSVVMKIRPNDFIPNVI (SEQIDNO: 53). Then, the genes encoding the above amino acid sequence were synthesized by Sangon Biotech (Shanghai) Co.,Ltd.; the FGL1 genes and the Fc segment genes of human IgG1 were fused together by recombinant PCR, and the above genes with a Fc tag were constructed into a mammalian expression vector; transiently transfected and expressed in HEK293E cells; after five days, the recombinant protein in the cell culture supernatant was purified by Protein A affinity chromatography, and the protein concentration was determined by ultraviolet spectrophotometry, the resulting protein was named FGL1-hFc. The biotin labeling method of FGL1-hFc is as follows: Biotin N-hydroxysuccinimide ester (Sigma/Cat. No.: H1759-100MG) was prepared into 100 mM mother liquor using anhydrous DMSO; the corresponding molar concentration was calculated according to the molecular weight and concentration of FGL1-hFc; An appropriate volume of Biotin N-hydroxysuccinimide ester solution was mixed with FGL1-hFc at a molar ratio of 20:1, and incubated at room temperature for 1 hour; after dialysis, the protein concentration was determined by ultraviolet spectrophotometry, and the resulting biotinylated protein was named Biotin-FGL1-hFc.

The method of constructing a CHO-S cell line stably expressing LAG-3 is as follows: CHO-S cells were purchased from Thermo Fisher Scientific. The full-length human LAG-3 gene was constructed into the pCHO1.0 expression vector; the expression vector was transfected into CHO-S cells with PEI (polyethylenimine) and selected with methotrexate and puromycin, and then single clone wells were selected by limiting dilution method, to obtain a CHO-S monoclonal cell line stably expressing human LAG-3, which was named CHO-S-LAG-3.

The method of detecting blocking of antibody on the interaction between FGL1 and LAG-3 by flow cytometry is described as follows:
CHO-S-LAG-3 cells were inoculated into 96-well plates (round-bottomed, with a lid) at 200,000 cells per well; centrifuged at 300 g for 5 min, the cell culture supernatant was aspirated with a pipettor, 200 µl PBS + 1% BSA (PBS solution containing 1% bovine serum albumin) was added to each well to resuspend the cells, then centrifuged at 300 g and the supernatant was aspirated; serially diluted anti-LAG-3 antibody and Biotin-FGLl-hFc at a constant concentration of 2 µg/ml were added and incubated at room temperature for 1 hour; centrifuged at 300 g, the supernatant was aspirated, and the cells was washed twice with PBS + 1% BSA; centrifuged at 300 g, the supernatant was aspirated; PE Streptavidin diluted 1:2000 with PBS + 1% BSA was added to each well at 200 µl per well, incubated at room temperature for 0.5 hours, centrifuged at 300 g, and the supernatant was aspirated, the cells were washed twice with PBS + 1% BSA; finally the cells were resuspended with 200 µl PBS. The fluorescence intensity of the cells in each well in the PE channel was measured on the flow cytometer. GraphPad Prism6 was used to analyze data and graph, and calculate EC50.

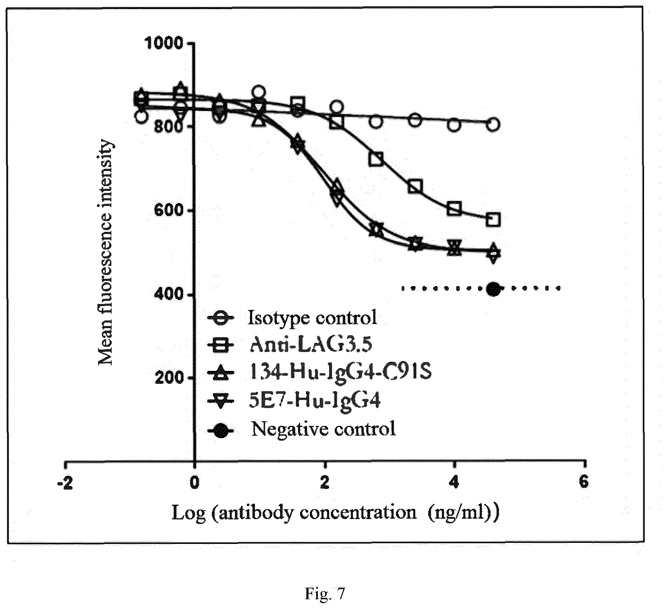

The experimental results are shown in Fig. 7. The IC50s of Anti-LAG3.5, 134-Hu-IgG4-C91S and 5E7-Hu-IgG4 to block the interaction between FGL1 and LAG-3 were 785.6 ng/ml, 97.57 ng/ml and 97.71 ng/ml, respectively, indicating that the blocking ability of 134-Hu-IgG4-C91S and 5E7-Hu-IgG4 is stronger than that of Anti-LAG3.5.

### Example 12. Inhibition of anti-human LAG-3 antibody on MC38 xenograft model in transgenic mice

The animal experimental procedures are as follows: Human LAG-3 transgenic mice (germline background: C57BL/6) and MC38 mouse colorectal cell line were provided by Shanghai Model Organisms Center. In the transgenic mice, the extracellular segment of mouse LAG-3 has been replaced by the homologous part of the human LAG-3 gene, therefore, the humanized anti-LAG-3 antibody of the present invention can recognize LAG-3 molecules in the transgenic mice.

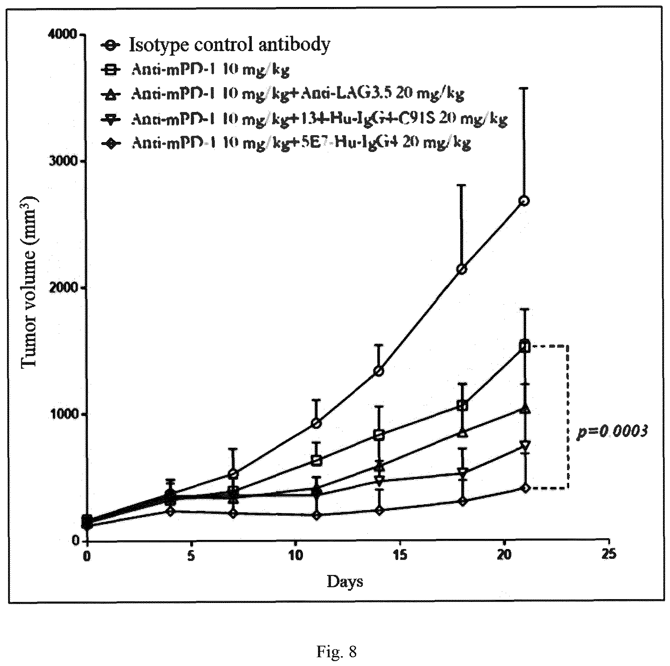

The specific implementation procedures are as follows: MC38 cells were cultured and expanded in vitro in DMEM medium containing 10% fetal bovine serum (fetal bovine serum and DMEM medium were purchased from Thermo Fisher Scientific); the expanded MC38 cells were inoculated in human LAG-3 transgenic mice, each mouse was inoculated with 1×10⁶ cells subcutaneously; when the tumor cells to be inoculated grew to a volume close to 100 mm³, the animals were randomly divided into five groups, 5 in each group: isotype control antibody group (injection of isotype control antibody only); Anti-mPD-1 (purchased from Bio X Cell, Cat. No.: BP0146) 10 mg/kg; Anti-mPD-1 10 mg/kg + Anti-LAG3.5 20 mg/kg; Anti-mPD-1 10 mg/kg + 134-Hu-IgG4-C91S 20 mg/kg; Anti-mPD-1 10 mg/kg + 5E7-Hu-IgG4 20 mg/kg. The mice were administered by intraperitoneal injection twice a week for three weeks according to the above dosing regimen. The tumor volume was measured and calculated twice a week. Finally, the tumor growth curve of each group is shown in Fig. 8. Anti-LAG-3 monoclonal antibody has weak anti-tumor effect when used as a single agent (Reference: Woo SR, Turnis ME, Goldberg MV, et al. Immune inhibitory molecules LAG-3 and PD-1 synergistically regulate T-cell function to promote tumoral immune escape[J]. Cancer research, 2012, 72(4): 917-927.). Considering animal welfare, single-agent group of anti-LAG-3 monoclonal antibody was not provided herein.

The experimental results show that, compared with the control group, Anti-mPD-1 can significantly inhibit the growth of MC38 tumors in mice (*P*=0.0249); compared with single-agent Anti-mPD-1, the combination of Anti-LAG3.5 and Anti-mPD-1 can further inhibit the growth of MC38 tumors, but there is no statistically significant difference (*P*=0.1174, *P*<0.05 was considered as a significant difference); compared with single-agent Anti-mPD-1, the combination of 134-Hu

-IgG4-C91S and Anti-mPD-1 can further inhibit the growth of MC38 tumors (*P*=0.0159); compared with single-agent Anti-mPD-1, the combination of 5E7-Hu-IgG4 and Anti-mPD-1 also can further inhibit the growth of MC38 tumors (*P*=0.0003).

## Claims

1. An antibody or an antigen-binding fragment thereof that binds to human LAG-3, **characterized in that**, human LAG-3 epitope to which the antibody or the antigen-bingding fragment binds comprises the following amino acid sequence: AAAPGHPLA (SEQ ID NO: 50).

2. The antibody or the antigen-binding fragment thereof that binds to human LAG-3 according to claim 1, **characterized in that**, the human LAG-3 epitope to which the antibody or the antigen-bingding fragment binds comprises the following amino acid sequence: GPPAAAPGHPLA (SEQ ID NO: 48) or AAAPGHPLAPGPHPAAPSS (SEQ ID NO: 49).

3. An antibody or an antigen-binding fragment thereof that binds to human LAG-3, **characterized in that**, the antibody or the antigen-binding fragment comprises:
(a) heavy chain complementarity-determining regions HCDR1, HCDR2, HCDR3, the HCDR1 having an amino acid sequence as shown in SEQ ID NO: 9, the HCDR2 having an amino acid sequence as shown in SEQ ID NO: 10, and the HCDR3 having an amino acid sequence as shown in SEQ ID NO: 11, and light chain complementarity-determining regions LCDR1, LCDR2, LCDR3, the LCDR1 having an amino acid sequence as shown in SEQ ID NO: 12, the LCDR2 having an amino acid sequence as shown in SEQ ID NO: 13, and the LCDR3 having an amino acid sequence as shown in SEQ ID NO: 14 or SEQ ID NO: 43; or
(b) heavy chain complementarity-determining regions HCDR1, HCDR2, HCDR3, the HCDR1 having an amino acid sequence as shown in SEQ ID NO: 15, the HCDR2 having an amino acid sequence as shown in SEQ ID NO: 16, and the HCDR3 having an amino acid sequence as shown in SEQ ID NO: 17, and light chain complementarity-determining regions LCDR1, LCDR2, LCDR3, the LCDR1 having an amino acid sequence as shown in SEQ ID NO: 18, the LCDR2 having an amino acid sequence as shown in SEQ ID NO: 19, and the LCDR3 having an amino acid sequence as shown in SEQ ID NO: 20.

4. The antibody or the antigen-binding fragment thereof that binds to human LAG-3 according to any one of claims 1-3, **characterized in that**, the antibody is a monoclonal antibody or a polyclonal antibody.

5. The antibody or the antigen-binding fragment thereof that binds to human LAG-3 according to claim 4, **characterized in that**, the antibody is a monoclonal antibody.

6. The antibody or the antigen-binding fragment thereof that binds to human LAG-3 according to any one of claims 1-3, **characterized in that**, the antibody is a murine antibody, a chimeric antibody or a humanized antibody.

7. The antibody or the antigen-binding fragment thereof that binds to human LAG-3 according to claim 6, **characterized in that**, the antibody is a humanized antibody.

8. The antibody or the antigen-binding fragment thereof that binds to human LAG-3 according to any one of claims 1-3, **characterized in that**, the antigen-binding fragment comprises a Fab fragment, a F(ab)'₂ fragment, a Fv fragment.

9. The antibody or the antigen-binding fragment thereof that binds to human LAG-3 according to any one of claims 1-3, **characterized in that**, the antibody or antigen-binding fragment thereof that binds to human LAG-3 comprises a heavy chain variable region and a light chain variable region, the heavy chain variable region and the light chain variable region being selected from the group consisting of:
(a) a heavy chain variable region having an amino acid sequence as shown in SEQ ID NO: 2, and a light chain variable region having an amino acid sequence as shown in SEQ ID NO: 4;
(b) a heavy chain variable region having an amino acid sequence as shown in SEQ ID NO: 6, and a light chain variable region having an amino acid sequence as shown in SEQ ID NO: 8;
(c) a heavy chain variable region having an amino acid sequence as shown in SEQ ID NO: 22, and a light chain variable region having an amino acid sequence as shown in SEQ ID NO: 24; and
(d) a heavy chain variable region having an amino acid sequence as shown in SEQ ID NO: 26, and a light chain variable region having an amino acid sequence as shown in SEQ ID NO: 28.

10. The antibody or the antigen-binding fragment thereof that binds to human LAG-3 according to claim 9, **characterized in that**, the antibody or antigen-binding fragment thereof that binds to human LAG-3 comprises a heavy chain constant region and a light chain constant region, the heavy chain constant region is selected from the group consisting of IgG1, IgG2, IgG3 and IgG4 heavy chain constant region, and the light chain constant region is selected from the group consisting of κ or λ light chain constant region.

11. The antibody or the antigen-binding fragment thereof that binds to human LAG-3 according to claim 10, **characterized in that**, the heavy chain constant region is IgG4 heavy chain constant region, and the light chain constant region is κ light chain constant region.

12. The antibody or the antigen-binding fragment thereof that binds to human LAG-3 according to claim 11, **characterized in that**, the heavy chain constant region has an amino acid sequence as shown in SEQ ID NO: 30, and the light chain constant region has an amino acid sequence as shown in SEQ ID NO: 34.

13. The antibody or antigen-binding fragment thereof that binds to human LAG-3 according to any one of claims 9-12, **characterized in that**, the antibody or antigen-binding fragment thereof that binds to human LAG-3 comprises a heavy chain and a light chain, the heavy chain and the light chain being selected from the group consisting of:
(a) a heavy chain having an amino acid sequence as shown in SEQ ID NO: 32, and a light chain having an amino acid sequence as shown in SEQ ID NO: 36; and
(b) a heavy chain having an amino acid sequence as shown in SEQ ID NO: 38, and a light chain having an amino acid sequence as shown in SEQ ID NO: 40.

14. A nucleotide sequence, **characterized in that**, the nucleotide sequence encodes the antibody or the antigen-binding fragment thereof that binds to human LAG-3 according to any one of claims 1-13.

15. The nucleotide sequence according to claim 14, **characterized in that**, the nucleotide sequence comprises:
(a) a nucleotide sequence encoding the heavy chain variable region as shown in SEQ ID NO: 1, and a nucleotide sequence encoding the light chain variable region as shown in SEQ ID NO: 3;
(b) a nucleotide sequence encoding the heavy chain variable region as shown in SEQ ID NO: 5, and a nucleotide sequence encoding the light chain variable region as shown in SEQ ID NO: 7;
(c) a nucleotide sequence encoding the heavy chain variable region as shown in SEQ ID NO: 21, and a nucleotide sequence encoding the light chain variable region as shown in SEQ ID NO: 23; or
(d) a nucleotide sequence encoding the heavy chain variable region as shown in SEQ ID NO: 25, and a nucleotide sequence encoding the light chain variable region as shown in SEQ ID NO: 27.

16. The nucleotide sequence according to claim 15, **characterized in that**, the nucleotide sequence comprises a nucleotide sequence encoding the heavy chain constant region as shown in SEQ ID NO: 29, and a nucleotide sequence encoding the light chain constant region as shown in SEQ ID NO: 33.

17. The nucleotide sequence according to any one of claims 14-16, **characterized in that**, the nucleotide sequence comprises:
(a) a nucleotide sequence encoding the heavy chain as shown in SEQ ID NO: 31, and a nucleotide sequence encoding the light chain as shown in SEQ ID NO: 35; or
(b) a nucleotide sequence encoding the heavy chain as shown in SEQ ID NO: 37, and a nucleotide sequence encoding the light chain as shown in SEQ ID NO: 39.

18. An expression vector, **characterized in that**, the expression vector comprises the nucleotide sequence according to any one of claims 14-17.

19. A host cell, **characterized in that**, the host cell comprises the expression vector according to claim 18.

20. A method of preparing the antibody or the antigen-binding fragment thereof that binds to human LAG-3 according to any one of claims 1-13, **characterized in that**, the method comprises the following steps of:
a) culturing the host cell according to claim 19 under expression conditions, to express the antibody or the antigen-binding fragment thereof that binds to human LAG-3;
b) isolating and purifying the antibody or the antigen-binding fragment thereof that binds to human LAG-3 obtained by step a).

21. A pharmaceutical composition, **characterized in that**, the pharmaceutical composition comprises the antibody or the antigen-binding fragment thereof that binds to human LAG-3 according to any one of claims 1-13 and a pharmaceutically acceptable carrier.

22. The pharmaceutical composition according to claim 21, **characterized in that**, the pharmaceutical composition further comprises a PD-1 inhibitor.

23. The pharmaceutical composition according to claim 22, **characterized in that**, the PD-1 inhibitor is an antibody or an antigen-binding fragment thereof that binds to PD-1.

24. Use of the antibody or the antigen-binding fragment thereof that binds to human LAG-3 according to any one of claims 1-13 or use of the pharmaceutical composition according to any one of claims 21-23 in the preparation of a drug for the treatment of cancers.

25. The use according to claim 24, **characterized in that**, the cancer is selected from the group consisting of melanoma, renal cell carcinoma, non-small cell lung cancer, classical Hodgkin's lymphoma, urothelial carcinoma, colorectal cancer and liver cancer.


SEQUENCE LISTING

<110>  SUNSHINE GUOJIAN PHARMACEUTICAL (SHANGHAI) CO., LTD.

<120>  ANTIBODY BINDING HUMAN LAG-3, AND PREPARATION METHOD AND USE THEREOF

<130>  SH363-20P450007

<160>  53

<170>  PatentIn version 3.5

<210>  1
<211>  360
<212>  DNA
<213>  Mus musculus

<400>  1
gaggtccagc tgcaacagtc tggacctgtg ctggtgaagc ctggggcttc agtaaagatg      60

tcctgtaagg cttctggata cacactcact gcctactata tgaactgggt gaagcaaagt     120

cgtggaaaga gccttgagtg gattggagtt attaatcctt acaacggtga ttctagctac     180

aaccagaagt tcaagggcaa ggccacattg actgttgaca agtcctccag cacagcctac     240

atggagctca acagcctgac atctgaggac tctgcagtct attactgtgc aagggatgat     300

ggttactacc gatggtactt cgatgtctgg ggcacaggga ccacggtcac cgtctcctca     360


<210>  2
<211>  120
<212>  PRT
<213>  Mus musculus

<400>  2

Glu Val Gln Leu Gln Gln Ser Gly Pro Val Leu Val Lys Pro Gly Ala
1               5                   10                  15


Ser Val Lys Met Ser Cys Lys Ala Ser Gly Tyr Thr Leu Thr Ala Tyr
            20                  25                  30


Tyr Met Asn Trp Val Lys Gln Ser Arg Gly Lys Ser Leu Glu Trp Ile
        35                  40                  45


Gly Val Ile Asn Pro Tyr Asn Gly Asp Ser Ser Tyr Asn Gln Lys Phe
    50                  55                  60


Lys Gly Lys Ala Thr Leu Thr Val Asp Lys Ser Ser Ser Thr Ala Tyr
65                  70                  75                  80


Met Glu Leu Asn Ser Leu Thr Ser Glu Asp Ser Ala Val Tyr Tyr Cys
                85                  90                  95


Ala Arg Asp Asp Gly Tyr Tyr Arg Trp Tyr Phe Asp Val Trp Gly Thr

            100                 105                 110

Gly Thr Thr Val Thr Val Ser Ser
        115                 120

<210> 3
<211> 321
<212> DNA
<213> Mus musculus

<400> 3
gacatccaga tgacccagtc tccatcctcc ttatctgcct ctctgggaga aagagtcagt 60

ctcacttgtc gggcaagtca ggacattggt agtagattaa actggcttca gcagggacca 120

gatggaagta ttaaacgcct gatctacgcc acatccagtt tagaatctgg tgtccccaaa 180

aggttcagtg gcagtaggtc tgggtcagat tactttctca ccatcagcag ccttgagtct 240

gaagattttg tagactatta ctgtctacag tgtggtagtt ctcctccgac gttcggtgga 300

ggcaccaagc tggaaatcaa a 321

<210> 4
<211> 107
<212> PRT
<213> Mus musculus

<400> 4

Asp Ile Gln Met Thr Gln Ser Pro Ser Ser Leu Ser Ala Ser Leu Gly
1               5                   10                  15

Glu Arg Val Ser Leu Thr Cys Arg Ala Ser Gln Asp Ile Gly Ser Arg
            20                  25                  30

Leu Asn Trp Leu Gln Gln Gly Pro Asp Gly Ser Ile Lys Arg Leu Ile
        35                  40                  45

Tyr Ala Thr Ser Ser Leu Glu Ser Gly Val Pro Lys Arg Phe Ser Gly
    50                  55                  60

Ser Arg Ser Gly Ser Asp Tyr Phe Leu Thr Ile Ser Ser Leu Glu Ser
65                  70                  75                  80

Glu Asp Phe Val Asp Tyr Tyr Cys Leu Gln Cys Gly Ser Ser Pro Pro
                85                  90                  95

Thr Phe Gly Gly Gly Thr Lys Leu Glu Ile Lys
            100                 105

<210> 5
<211> 348

<212>  DNA
<213>  Mus musculus

<400>  5
gaagtgaagc tggtggagtc tgggggaggc ttagtgaagc ctggagggtc cctgaaactc      60

tcctgtgcag cctctggatt cactttcagt gacgattaca tggcttggtt tcgccagact     120

cctgagaaaa ggctggagtg ggtcgcatcc attagtcatg gtggtgatta catctactat     180

gcagacaatt tgaaggggcg attcaccatc tccagagaca atgccaagaa caccctgtac     240

ctgcaaatga gcagtctgaa gtctgaggac acagccatat atttctgttc aagagatcgg     300

cgatccattg actactgggg ccaaggcacc actctcacag tctcctca                  348


<210>  6
<211>  116
<212>  PRT
<213>  Mus musculus

<400>  6

Glu Val Lys Leu Val Glu Ser Gly Gly Gly Leu Val Lys Pro Gly Gly
1               5                   10                  15


Ser Leu Lys Leu Ser Cys Ala Ala Ser Gly Phe Thr Phe Ser Asp Asp
            20                  25                  30


Tyr Met Ala Trp Phe Arg Gln Thr Pro Glu Lys Arg Leu Glu Trp Val
        35                  40                  45


Ala Ser Ile Ser His Gly Gly Asp Tyr Ile Tyr Tyr Ala Asp Asn Leu
    50                  55                  60


Lys Gly Arg Phe Thr Ile Ser Arg Asp Asn Ala Lys Asn Thr Leu Tyr
65                  70                  75                  80


Leu Gln Met Ser Ser Leu Lys Ser Glu Asp Thr Ala Ile Tyr Phe Cys
                85                  90                  95


Ser Arg Asp Arg Arg Ser Ile Asp Tyr Trp Gly Gln Gly Thr Thr Leu
            100                 105                 110


Thr Val Ser Ser
        115


<210>  7
<211>  321
<212>  DNA
<213>  Mus musculus

<400>  7
gatatccaga tgacacagat tacatcctcc ctgtctgcct ctctgggaga cagagtcacc      60

atcacttgca gggcaagtca ggacattagc aattatttaa gctggtatca gcagaaacca     120

gatggaacta ttaaactcct gatctactac acatcaagat tacactcagg agtcccatca     180

aggttcagtg gcagtgggtc tggaacagat tattctctca ccattagcga cctggagcaa     240

gaagatatcg ccacttactt ttgccaacag ggtaaaacgc ttccgtacac gttcggaggg     300

gggaccaagc tggaaagaaa a                                               321

<210>  8
<211>  107
<212>  PRT
<213>  Mus musculus

<400>  8

Asp Ile Gln Met Thr Gln Ile Thr Ser Ser Leu Ser Ala Ser Leu Gly
1               5                   10                  15

Asp Arg Val Thr Ile Thr Cys Arg Ala Ser Gln Asp Ile Ser Asn Tyr
            20                  25                  30

Leu Ser Trp Tyr Gln Gln Lys Pro Asp Gly Thr Ile Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asp Leu Glu Gln
65                  70                  75                  80

Glu Asp Ile Ala Thr Tyr Phe Cys Gln Gln Gly Lys Thr Leu Pro Tyr
                85                  90                  95

Thr Phe Gly Gly Gly Thr Lys Leu Glu Arg Lys
            100                 105

<210>  9
<211>  5
<212>  PRT
<213>  Mus musculus

<400>  9

Ala Tyr Tyr Met Asn
1               5

<210>  10
<211>  17
<212>  PRT
<213>  Mus musculus

<400>  10

Val Ile Asn Pro Tyr Asn Gly Asp Ser Ser Tyr Asn Gln Lys Phe Lys
1               5                   10                  15

Gly

<210>  11
<211>  11
<212>  PRT
<213>  Mus musculus

<400>  11

Asp Asp Gly Tyr Tyr Arg Trp Tyr Phe Asp Val
1               5                   10

<210>  12
<211>  11
<212>  PRT
<213>  Mus musculus

<400>  12

Arg Ala Ser Gln Asp Ile Gly Ser Arg Leu Asn
1               5                   10

<210>  13
<211>  7
<212>  PRT
<213>  Mus musculus

<400>  13

Ala Thr Ser Ser Leu Glu Ser
1               5

<210>  14
<211>  9
<212>  PRT
<213>  Mus musculus

<400>  14

Leu Gln Cys Gly Ser Ser Pro Pro Thr
1               5

<210>  15
<211>  5
<212>  PRT
<213>  Mus musculus

<400>  15

Asp Asp Tyr Met Ala
1               5

<210>  16
<211>  17
<212>  PRT
<213>  Mus musculus

<400>  16

Ser Ile Ser His Gly Gly Asp Tyr Ile Tyr Tyr Ala Asp Asn Leu Lys
1               5                   10                  15

Gly

<210>  17
<211>  7
<212>  PRT
<213>  Mus musculus

<400>  17

Asp Arg Arg Ser Ile Asp Tyr
1               5

<210>  18
<211>  11
<212>  PRT
<213>  Mus musculus

<400>  18

Arg Ala Ser Gln Asp Ile Ser Asn Tyr Leu Ser
1               5                   10

<210>  19
<211>  7
<212>  PRT
<213>  Mus musculus

<400>  19

Tyr Thr Ser Arg Leu His Ser
1               5

<210>  20
<211>  9
<212>  PRT
<213>  Mus musculus

<400>  20

Gln Gln Gly Lys Thr Leu Pro Tyr Thr
1               5

<210>  21
<211>  360

<212>  DNA
<213>  Synthetic

<400>  21
caagtgcagc tggtgcagtc cggcgccgaa gtgaagaagc ctggcgcctc cgtgaaggtg      60

agctgcaagg cttccggcta caccctgacc gcctactaca tgaactgggt gaggcaggcc     120

cctggccagt ccctggaatg gatcggcgtg atcaacccct acaacggcga ctcctcctat     180

aaccagaagt tcaagggccg ggccaccctg accgtggaca agtccaccag caccgcctac     240

atggagctgt cctccctgag gtccgaggac accgccgtct actactgcgc cagggacgac      300

ggctactaca ggtggtactt cgacgtgtgg ggccagggca ccctggtgac agtgtcctcc     360


<210>  22
<211>  120
<212>  PRT
<213>  Synthetic

<400>  22

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Leu Thr Ala Tyr
            20                  25                  30


Tyr Met Asn Trp Val Arg Gln Ala Pro Gly Gln Ser Leu Glu Trp Ile
        35                  40                  45


Gly Val Ile Asn Pro Tyr Asn Gly Asp Ser Ser Tyr Asn Gln Lys Phe
    50                  55                  60


Lys Gly Arg Ala Thr Leu Thr Val Asp Lys Ser Thr Ser Thr Ala Tyr
65                  70                  75                  80


Met Glu Leu Ser Ser Leu Arg Ser Glu Asp Thr Ala Val Tyr Tyr Cys
                85                  90                  95


Ala Arg Asp Asp Gly Tyr Tyr Arg Trp Tyr Phe Asp Val Trp Gly Gln
            100                 105                 110


Gly Thr Leu Val Thr Val Ser Ser
        115                 120


<210>  23
<211>  321
<212>  DNA
<213>  Synthetic

<400>  23
gacatccaga tgacccagtc cccttcctcc ctgtccgctt ccgtgggcga caggtgacc       60

atcacctgta gggcctccca ggacatcggc tccaggctga actggctgca gcagaagccc 120

ggcaagtcca tcaagcggct gatctacgcc acctcctccc tggagtccgg cgtgccttcc 180

aggttctccg gctccaggtc cggctccgac tacaccctga ccatctcctc cctgcagccc 240

gaggacttcg ccacctacta ctgcctgcag tccggctcct cccctcctac cttcggccag 300

ggcaccaagg tggagatcaa g 321

<210> 24
<211> 107
<212> PRT
<213> Synthetic

<400> 24

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Ser Arg
            20                  25                  30

Leu Asn Trp Leu Gln Gln Lys Pro Gly Lys Ser Ile Lys Arg Leu Ile
        35                  40                  45

Tyr Ala Thr Ser Ser Leu Glu Ser Gly Val Pro Ser Arg Phe Ser Gly
    50                  55                  60

Ser Arg Ser Gly Ser Asp Tyr Thr Leu Thr Ile Ser Ser Leu Gln Pro
65                  70                  75                  80

Glu Asp Phe Ala Thr Tyr Tyr Cys Leu Gln Ser Gly Ser Ser Pro Pro
                85                  90                  95

Thr Phe Gly Gln Gly Thr Lys Val Glu Ile Lys
            100                 105

<210> 25
<211> 348
<212> DNA
<213> Synthetic

<400> 25
gaagtgcagc tggtggagtc cggaggcgga ctggtgaagc ctggaggctc cctgaggctg 60

tcctgtgccg cttccggctt caccttctcc gacgactaca tggcctggtt caggcaggcc 120

cctggaaaga ggctggagtg ggtggcttcc atctcccacg gcggcgacta catctactac 180

gccgacaacc tgaagggcag gttcaccatc tccagggaca acgccaagaa ctccctgtac 240

ctgcagatga actccctgag ggccgaggac accgccgtgt acttctgctc cagggacagg 300

aggtccatcg actattgggg ccagggcacc ctggtgacag tgtcctcc 348

<210> 26
<211> 116
<212> PRT
<213> Synthetic

<400> 26

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Asp
            20                  25                  30

Tyr Met Ala Trp Phe Arg Gln Ala Pro Gly Lys Arg Leu Glu Trp Val
        35                  40                  45

Ala Ser Ile Ser His Gly Gly Asp Tyr Ile Tyr Tyr Ala Asp Asn Leu
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ser Arg Asp Arg Arg Ser Ile Asp Tyr Trp Gly Gln Gly Thr Leu Val
            100                 105                 110

Thr Val Ser Ser
        115

<210> 27
<211> 321
<212> DNA
<213> Synthetic

<400> 27
   gacatccaga tgacccagtc cccttcctcc ctgtccgctt ccgtgggcga cagggtgacc 60
   atcacctgca gggccagcca ggacatctcc aactacctgt cctggtacca gcagaagccc 120
   ggcaagacca tcaagctgct gatctactac acctccaggc tgcacagcgg cgtgcctagc 180
   aggttctccg gttctggctc cggcaccgac tacaccttca ccatcagctc cctgcagccc 240
   gaggacatcg ccacctactt ctgccagcag ggcaagaccc tgccctacac cttcggccag 300
   ggcaccaagg tggagatcaa g 321

<210> 28
<211> 107

<212>  PRT
<213>  Synthetic

<400>  28

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Asn Tyr
            20                  25                  30

Leu Ser Trp Tyr Gln Gln Lys Pro Gly Lys Thr Ile Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Phe Cys Gln Gln Gly Lys Thr Leu Pro Tyr
                85                  90                  95

Thr Phe Gly Gln Gly Thr Lys Val Glu Ile Lys
            100                 105

<210>  29
<211>  981
<212>  DNA
<213>  Homo

<400>  29
gcaagtacca agggacctag tgttttccct cttgcacctt gctccaggtc aacatcagag      60

tccacagctg ctcttggatg tctcgttaag gactacttcc cagagccagt taccgtatcc     120

tggaactccg gagctttgac aagcggcgtt catacattcc cagctgtgtt gcagagttct     180

gggttgtaca gtttgagctc agtggtgacc gtgccttcat cttctttggg cactaagacc     240

tacacctgca acgtggatca caagccaagc aacaccaagg tggataagag ggtggagtcc     300

aagtacggcc caccatgtcc tccatgtcca gcccctgaat ttttgggcgg gccttctgtc     360

tttctgtttc ctcctaaacc taaagatacc ctgatgatca gccgcacacc cgaagtcact     420

tgtgtggtcg tggatgtgtc tcaggaagat cccgaagtgc agtttaactg gtatgtcgat     480

ggcgtggaag tgcataatgc caaaactaag ccccgcgaag aacagttcaa cagcacttat     540

cgggtcgtgt ctgtgctcac agtcctccat caggattggc tgaatggaaa agaatataag     600

tgcaaggtga gcaataaggg cctccccagc agcatcgaga agactattag caaagccaaa     660

gggcagccac gggaacccca ggtgtacact ctgcccccct ctcaggagga gatgactaaa     720

aatcaggtct ctctgacttg tctggtgaaa gggttttatc ccagcgacat tgccgtggag 780

tgggagtcta atggccagcc cgagaataat tataagacaa ctccccccgt cctggactct 840

gacggcagct ttttcctgta ttctcggctg acagtggaca aaagtcgctg gcaggagggc 900

aatgtcttta gttgcagtgt catgcatgag gccctgcaca atcactatac acagaaaagc 960

ctgtctctga gtctgggcaa a 981

<210> 30
<211> 327
<212> PRT
<213> Homo

<400> 30

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp

180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  31
<211>  1341
<212>  DNA
<213>  Synthetic

<400>  31
caagtgcagc tggtgcagtc cggcgccgaa gtgaagaagc ctggcgcctc cgtgaaggtg      60

agctgcaagg cttccggcta caccctgacc gcctactaca tgaactgggt gaggcaggcc     120

cctggccagt ccctggaatg gatcggcgtg atcaacccct acaacggcga ctcctcctat     180

aaccagaagt tcaagggccg ggccaccctg accgtggaca agtccaccag caccgcctac     240

atggagctgt cctccctgag gtccgaggac accgccgtct actactgcgc cagggacgac     300

ggctactaca ggtggtactt cgacgtgtgg ggccagggca ccctggtgac agtgtcctcc     360

gcaagtacca agggacctag tgttttccct cttgcacctt gctccaggtc aacatcagag     420

tccacagctg ctcttggatg tctcgttaag gactacttcc cagagccagt taccgtatcc     480

tggaactccg gagctttgac aagcggcgtt catacattcc cagctgtgtt gcagagttct     540

gggttgtaca gtttgagctc agtggtgacc gtgccttcat cttctttggg cactaagacc 600

tacacctgca acgtggatca caagccaagc aacaccaagg tggataagag ggtggagtcc 660

aagtacggcc caccatgtcc tccatgtcca gcccctgaat ttttgggcgg gccttctgtc 720

tttctgtttc ctcctaaacc taaagatacc ctgatgatca gccgcacacc cgaagtcact 780

tgtgtggtcg tggatgtgtc tcaggaagat cccgaagtgc agtttaactg gtatgtcgat 840

ggcgtggaag tgcataatgc caaaactaag ccccgcgaag aacagttcaa cagcacttat 900

cgggtcgtgt ctgtgctcac agtcctccat caggattggc tgaatgggaa agaatataag 960

tgcaaggtga gcaataaggg cctccccagc agcatcgaga agactattag caaagccaaa 1020

gggcagccac gggaacccca ggtgtacact ctgcccccct ctcaggagga gatgactaaa 1080

aatcaggtct ctctgacttg tctggtgaaa gggttttatc ccagcgacat tgccgtggag 1140

tgggagtcta atggccagcc cgagaataat tataagacaa ctccccccgt cctggactct 1200

gacggcagct ttttcctgta ttctcggctg acagtggaca aaagtcgctg gcaggagggc 1260

aatgtcttta gttgcagtgt catgcatgag gccctgcaca atcactatac acagaaaagc 1320

ctgtctctga gtctgggcaa a 1341

<210> 32
<211> 447
<212> PRT
<213> Synthetic

<400> 32

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Leu Thr Ala Tyr
            20                  25                  30

Tyr Met Asn Trp Val Arg Gln Ala Pro Gly Gln Ser Leu Glu Trp Ile
        35                  40                  45

Gly Val Ile Asn Pro Tyr Asn Gly Asp Ser Ser Tyr Asn Gln Lys Phe
    50                  55                  60

Lys Gly Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Asp Gly Tyr Tyr Arg Trp Tyr Phe Asp Val Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445

<210>  33
<211>  321
<212>  DNA
<213>  Homo

<400>  33
cgcactgtgg ctgcccccag tgttttcata tttcccccca gtgatgagca actgaagtcc      60

ggcacagcct ctgttgtatg tctgctgaat aatttttatc cacgggaggc caaggtgcag     120

tggaaggtgg acaatgccct gcagtctggg aactctcaag agagtgtgac agagcaggac     180

agtaaagaca gcacctatag cctcagcagc accctgaccc tgtctaaagc cgactatgaa     240

aaacacaaag tgtatgcctg cgaagtgacc catcaggggc tcagctctcc cgttaccaag     300

agctttaacc gaggcgaatg t                                               321

<210>  34
<211>  107
<212>  PRT
<213>  Homo

<400>  34

<210>  35
<211>  642
<212>  DNA
<213>  Synthetic

<400>  35
gacatccaga tgacccagtc cccttcctcc ctgtccgctt ccgtgggcga cagggtgacc      60

atcacctgta gggcctccca ggacatcggc tccaggctga actggctgca gcagaagccc     120

ggcaagtcca tcaagcggct gatctacgcc acctcctccc tggagtccgg cgtgccttcc     180

aggttctccg gctccaggtc cggctccgac tacaccctga ccatctcctc cctgcagccc     240

gaggacttcg ccacctacta ctgcctgcag tccggctcct cccctcctac cttcggccag     300

ggcaccaagg tggagatcaa gcgcactgtg gctgccccca gtgttttcat atttcccccc     360

agtgatgagc aactgaagtc cggcacagcc tctgttgtat gtctgctgaa taatttttat     420

ccacgggagg ccaaggtgca gtggaaggtg gacaatgccc tgcagtctgg gaactctcaa     480

gagagtgtga cagagcagga cagtaaagac agcacctata gcctcagcag caccctgacc     540

ctgtctaaag ccgactatga aaaacacaaa gtgtatgcct gcgaagtgac ccatcagggg     600

ctcagctctc ccgttaccaa gagctttaac cgaggcgaat gt                        642

<210>  36
<211>  214
<212>  PRT
<213>  Synthetic

<400>  36

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Ser Arg
            20                  25                  30

Leu Asn Trp Leu Gln Gln Lys Pro Gly Lys Ser Ile Lys Arg Leu Ile
        35                  40                  45

Tyr Ala Thr Ser Ser Leu Glu Ser Gly Val Pro Ser Arg Phe Ser Gly
    50                  55                  60

Ser Arg Ser Gly Ser Asp Tyr Thr Leu Thr Ile Ser Ser Leu Gln Pro
65                  70                  75                  80

Glu Asp Phe Ala Thr Tyr Tyr Cys Leu Gln Ser Gly Ser Ser Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  37
<211>  1329
<212>  DNA
<213>  Synthetic

<400>  37
gaagtgcagc tggtggagtc cggaggcgga ctggtgaagc ctggaggctc cctgaggctg      60

tcctgtgccg cttccggctt caccttctcc gacgactaca tggcctggtt caggcaggcc     120

cctggaaaga ggctggagtg ggtggcttcc atctcccacg gcggcgacta catctactac     180

gccgacaacc tgaagggcag gttcaccatc tccagggaca acgccaagaa ctccctgtac     240

ctgcagatga actccctgag ggccgaggac accgccgtgt acttctgctc cagggacagg     300

aggtccatcg actattgggg ccagggcacc ctggtgacag tgtcctccgc aagtaccaag     360

ggacctagtg ttttccctct tgcaccttgc tccaggtcaa catcagagtc cacagctgct     420

cttggatgtc tcgttaagga ctacttccca gagccagtta ccgtatcctg gaactccgga     480

gctttgacaa gcggcgttca tacattccca gctgtgttgc agagttctgg gttgtacagt 540

ttgagctcag tggtgaccgt gccttcatct tctttgggca ctaagaccta cacctgcaac 600

gtggatcaca agccaagcaa caccaaggtg gataagaggg tggagtccaa gtacggccca 660

ccatgtcctc catgtccagc ccctgaattt ttgggcgggc cttctgtctt tctgtttcct 720

cctaaaccta aagataccct gatgatcagc cgcacacccg aagtcacttg tgtggtcgtg 780

gatgtgtctc aggaagatcc cgaagtgcag tttaactggt atgtcgatgg cgtggaagtg 840

cataatgcca aaactaagcc ccgcgaagaa cagttcaaca gcacttatcg ggtcgtgtct 900

gtgctcacag tcctccatca ggattggctg aatgggaaag aatataagtg caaggtgagc 960

aataagggcc tccccagcag catcgagaag actattagca aagccaaagg gcagccacgg 1020

gaaccccagg tgtacactct gcccccctct caggaggaga tgactaaaaa tcaggtctct 1080

ctgacttgtc tggtgaaagg gttttatccc agcgacattg ccgtggagtg ggagtctaat 1140

ggccagcccg agaataatta taagacaact ccccccgtcc tggactctga cggcagcttt 1200

ttcctgtatt ctcggctgac agtggacaaa agtcgctggc aggagggcaa tgtctttagt 1260

tgcagtgtca tgcatgaggc cctgcacaat cactatacac agaaaagcct gtctctgagt 1320

ctgggcaaa 1329

<210> 38
<211> 443
<212> PRT
<213> Synthetic

<400> 38

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Asp
            20                  25                  30

Tyr Met Ala Trp Phe Arg Gln Ala Pro Gly Lys Arg Leu Glu Trp Val
        35                  40                  45

Ala Ser Ile Ser His Gly Gly Asp Tyr Ile Tyr Tyr Ala Asp Asn Leu
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ser Arg Asp Arg Arg Ser Ile Asp Tyr Trp Gly Gln Gly Thr Leu Val
        100                 105                 110

Thr Val Ser Ser Ala Ser Thr Lys Gly Pro Ser Val Phe Pro Leu Ala
    115                 120                 125

Pro Cys Ser Arg Ser Thr Ser Glu Ser Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Lys Thr Tyr Thr Cys Asn Val Asp His Lys Pro Ser Asn Thr
        195                 200                 205

Lys Val Asp Lys Arg Val Glu Ser Lys Tyr Gly Pro Pro Cys Pro Pro
    210                 215                 220

Cys Pro Ala Pro Glu Phe Leu Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser Gln Glu Asp Pro Glu Val Gln Phe Asn
            260                 265                 270

Trp Tyr Val Asp Gly Val Glu Val His Asn Ala Lys Thr Lys Pro Arg
        275                 280                 285

Glu Glu Gln Phe Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Gly Leu Pro Ser Ser Ile Glu Lys Thr Ile Ser Lys Ala Lys
                325                 330                 335

Gly Gln Pro Arg Glu Pro Gln Val Tyr Thr Leu Pro Pro Ser Gln Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Arg Leu Thr Val Asp Lys Ser Arg Trp Gln Glu Gly
                405                 410                 415

Asn Val Phe Ser Cys Ser Val Met His Glu Ala Leu His Asn His Tyr
            420                 425                 430

Thr Gln Lys Ser Leu Ser Leu Ser Leu Gly Lys
        435                 440

<210>  39
<211>  642
<212>  DNA
<213>  Synthetic

<400>  39
gacatccaga tgacccagtc cccttcctcc ctgtccgctt ccgtgggcga cagggtgacc      60

atcacctgca gggccagcca ggacatctcc aactacctgt cctggtacca gcagaagccc     120

ggcaagacca tcaagctgct gatctactac acctccaggc tgcacagcgg cgtgcctagc     180

aggttctccg gttctggctc cggcaccgac tacaccttca ccatcagctc cctgcagccc     240

gaggacatcg ccacctactt ctgccagcag ggcaagaccc tgccctacac cttcggccag     300

ggcaccaagg tggagatcaa gcgcactgtg gctgccccca gtgttttcat atttcccccc     360

agtgatgagc aactgaagtc cggcacagcc tctgttgtat gtctgctgaa taatttttat     420

ccacgggagg ccaaggtgca gtggaaggtg gacaatgccc tgcagtctgg gaactctcaa     480

gagagtgtga cagagcagga cagtaaagac agcacctata gcctcagcag caccctgacc     540

ctgtctaaag ccgactatga aaaacacaaa gtgtatgcct gcgaagtgac ccatcagggg     600

ctcagctctc ccgttaccaa gagctttaac cgaggcgaat gt                        642

<210>  40
<211>  214
<212>  PRT
<213>  Synthetic

<400>  40

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Asn Tyr
            20                  25                  30

Leu Ser Trp Tyr Gln Gln Lys Pro Gly Lys Thr Ile Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Phe Cys Gln Gln Gly Lys Thr Leu Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  41
<211>  422
<212>  PRT
<213>  Homo

<400>  41

Val Pro Val Val Trp Ala Gln Glu Gly Ala Pro Ala Gln Leu Pro Cys
1               5                   10                  15

Ser Pro Thr Ile Pro Leu Gln Asp Leu Ser Leu Leu Arg Arg Ala Gly
            20                  25                  30

Val Thr Trp Gln His Gln Pro Asp Ser Gly Pro Pro Ala Ala Ala Pro
        35                  40                  45

Gly His Pro Leu Ala Pro Gly Pro His Pro Ala Ala Pro Ser Ser Trp
    50                  55                  60

Gly Pro Arg Pro Arg Arg Tyr Thr Val Leu Ser Val Gly Pro Gly Gly
65                  70                  75                  80

Leu Arg Ser Gly Arg Leu Pro Leu Gln Pro Arg Val Gln Leu Asp Glu
                85                  90                  95

Arg Gly Arg Gln Arg Gly Asp Phe Ser Leu Trp Leu Arg Pro Ala Arg
            100                 105                 110

Arg Ala Asp Ala Gly Glu Tyr Arg Ala Ala Val His Leu Arg Asp Arg
        115                 120                 125

Ala Leu Ser Cys Arg Leu Arg Leu Arg Leu Gly Gln Ala Ser Met Thr
    130                 135                 140

Ala Ser Pro Pro Gly Ser Leu Arg Ala Ser Asp Trp Val Ile Leu Asn
145                 150                 155                 160

Cys Ser Phe Ser Arg Pro Asp Arg Pro Ala Ser Val His Trp Phe Arg
                165                 170                 175

Asn Arg Gly Gln Gly Arg Val Pro Val Arg Glu Ser Pro His His His
            180                 185                 190

Leu Ala Glu Ser Phe Leu Phe Leu Pro Gln Val Ser Pro Met Asp Ser
        195                 200                 205

Gly Pro Trp Gly Cys Ile Leu Thr Tyr Arg Asp Gly Phe Asn Val Ser
    210                 215                 220

Ile Met Tyr Asn Leu Thr Val Leu Gly Leu Glu Pro Pro Thr Pro Leu
225                 230                 235                 240

Thr Val Tyr Ala Gly Ala Gly Ser Arg Val Gly Leu Pro Cys Arg Leu
                245                 250                 255

Pro Ala Gly Val Gly Thr Arg Ser Phe Leu Thr Ala Lys Trp Thr Pro

260                 265                 270

Pro Gly Gly Gly Pro Asp Leu Leu Val Thr Gly Asp Asn Gly Asp Phe
        275                 280                 285

Thr Leu Arg Leu Glu Asp Val Ser Gln Ala Gln Ala Gly Thr Tyr Thr
    290                 295                 300

Cys His Ile His Leu Gln Glu Gln Gln Leu Asn Ala Thr Val Thr Leu
305                 310                 315                 320

Ala Ile Ile Thr Val Thr Pro Lys Ser Phe Gly Ser Pro Gly Ser Leu
                325                 330                 335

Gly Lys Leu Leu Cys Glu Val Thr Pro Val Ser Gly Gln Glu Arg Phe
            340                 345                 350

Val Trp Ser Ser Leu Asp Thr Pro Ser Gln Arg Ser Phe Ser Gly Pro
        355                 360                 365

Trp Leu Glu Ala Gln Glu Ala Gln Leu Leu Ser Gln Pro Trp Gln Cys
    370                 375                 380

Gln Leu Tyr Gln Gly Glu Arg Leu Leu Gly Ala Ala Val Tyr Phe Thr
385                 390                 395                 400

Glu Leu Ser Ser Pro Gly Ala Gln Arg Ser Gly Arg Ala Pro Gly Ala
                405                 410                 415

Leu Pro Ala Gly His Leu
            420

<210>  42
<211>  17
<212>  PRT
<213>  Synthetic

<400>  42

Met Gly Val Lys Val Leu Phe Ala Leu Ile Cys Ile Ala Val Ala Glu
1               5                   10                  15

Ala

<210>  43
<211>  9
<212>  PRT
<213>  Synthetic

<400>  43

Leu Gln Ser Gly Ser Ser Pro Pro Thr
1               5

<210>  44
<211>  10
<212>  PRT
<213>  Synthetic

<400>  44

Trp Gly Gln Gly Thr Lys Val Glu Ile Lys
1               5                   10

<210>  45
<211>  10
<212>  PRT
<213>  Synthetic

<400>  45

Phe Gly Gln Gly Thr Lys Val Glu Ile Lys
1               5                   10

<210>  46
<211>  30
<212>  PRT
<213>  Homo

<400>  46

Gly Pro Pro Ala Ala Ala Pro Gly His Pro Leu Ala Pro Gly Pro His
1               5                   10                  15

Pro Ala Ala Pro Ser Ser Trp Gly Pro Arg Pro Arg Arg Tyr
            20                  25                  30

<210>  47
<211>  9
<212>  PRT
<213>  Homo

<400>  47

Pro His Pro Ala Ala Pro Ser Ser Trp
1               5

<210>  48
<211>  12
<212>  PRT
<213>  Homo

<400>  48

Gly Pro Pro Ala Ala Ala Pro Gly His Pro Leu Ala
1               5                   10

<210>  49
<211>  19
<212>  PRT
<213>  Homo

<400>  49

Ala Ala Ala Pro Gly His Pro Leu Ala Pro Gly Pro His Pro Ala Ala
1               5                   10                  15

Pro Ser Ser

<210>  50
<211>  9
<212>  PRT
<213>  Homo

<400>  50

Ala Ala Ala Pro Gly His Pro Leu Ala
1               5

<210>  51
<211>  232
<212>  PRT
<213>  Homo

<400>  51

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  52
<211>  233
<212>  PRT
<213>  Staphylococcus

<400>  52

Ser Glu Lys Ser Glu Glu Ile Asn Glu Lys Asp Leu Arg Lys Lys Ser
1               5                   10                  15

Glu Leu Gln Gly Thr Ala Leu Gly Asn Leu Lys Gln Ile Tyr Tyr Tyr
            20                  25                  30

Asn Glu Lys Ala Lys Thr Glu Asn Lys Glu Ser His Asp Gln Phe Leu
        35                  40                  45

Gln His Thr Ile Leu Phe Lys Gly Phe Phe Thr Asp His Ser Trp Tyr
    50                  55                  60

Asn Asp Leu Leu Val Asp Phe Asp Ser Lys Asp Ile Val Asp Lys Tyr
65                  70                  75                  80

Lys Gly Lys Lys Val Asp Leu Tyr Gly Ala Tyr Tyr Gly Tyr Gln Cys
                85                  90                  95

Ala Gly Gly Thr Pro Asn Lys Thr Ala Cys Met Tyr Gly Gly Val Thr
        100                 105                 110

Leu His Asp Asn Asn Arg Leu Thr Glu Glu Lys Lys Val Pro Ile Asn
        115                 120                 125

Leu Trp Leu Asp Gly Lys Gln Asn Thr Val Pro Leu Glu Thr Val Lys
    130                 135                 140

Thr Asn Lys Lys Asn Val Thr Val Gln Glu Leu Asp Leu Gln Ala Arg
145                 150                 155                 160

Arg Tyr Leu Gln Glu Lys Tyr Asn Leu Tyr Asn Ser Asp Val Phe Asp
                165                 170                 175

Gly Lys Val Gln Arg Gly Leu Ile Val Phe His Thr Ser Thr Glu Pro
            180                 185                 190

Ser Val Asn Tyr Asp Leu Phe Gly Ala Gln Gly Gln Tyr Ser Asn Thr
        195                 200                 205

Leu Leu Arg Ile Tyr Arg Asp Asn Lys Thr Ile Asn Ser Glu Asn Met
    210                 215                 220

His Ile Asp Ile Tyr Leu Tyr Thr Ser
225                 230

<210>  53
<211>  290
<212>  PRT
<213>  Homo

<400>  53

Leu Glu Asp Cys Ala Gln Glu Gln Met Arg Leu Arg Ala Gln Val Arg
1               5                   10                  15

Leu Leu Glu Thr Arg Val Lys Gln Gln Gln Val Lys Ile Lys Gln Leu
            20                  25                  30

Leu Gln Glu Asn Glu Val Gln Phe Leu Asp Lys Gly Asp Glu Asn Thr
        35                  40                  45

Val Ile Asp Leu Gly Ser Lys Arg Gln Tyr Ala Asp Cys Ser Glu Ile
    50                  55                  60

Phe Asn Asp Gly Tyr Lys Leu Ser Gly Phe Tyr Lys Ile Lys Pro Leu
65                  70                  75                  80

Gln Ser Pro Ala Glu Phe Ser Val Tyr Cys Asp Met Ser Asp Gly Gly
                85                  90                  95

Gly Trp Thr Val Ile Gln Arg Arg Ser Asp Gly Ser Glu Asn Phe Asn
            100                 105                 110

Arg Gly Trp Lys Asp Tyr Glu Asn Gly Phe Gly Asn Phe Val Gln Lys
        115                 120                 125

His Gly Glu Tyr Trp Leu Gly Asn Lys Asn Leu His Phe Leu Thr Thr
    130                 135                 140

Gln Glu Asp Tyr Thr Leu Lys Ile Asp Leu Ala Asp Phe Glu Lys Asn
145                 150                 155                 160

Ser Arg Tyr Ala Gln Tyr Lys Asn Phe Lys Val Gly Asp Glu Lys Asn
                165                 170                 175

Phe Tyr Glu Leu Asn Ile Gly Glu Tyr Ser Gly Thr Ala Gly Asp Ser
            180                 185                 190

Leu Ala Gly Asn Phe His Pro Glu Val Gln Trp Trp Ala Ser His Gln
        195                 200                 205

Arg Met Lys Phe Ser Thr Trp Asp Arg Asp His Asp Asn Tyr Glu Gly
    210                 215                 220

Asn Cys Ala Glu Glu Asp Gln Ser Gly Trp Trp Phe Asn Arg Cys His
225                 230                 235                 240

Ser Ala Asn Leu Asn Gly Val Tyr Tyr Ser Gly Pro Tyr Thr Ala Lys
                245                 250                 255

Thr Asp Asn Gly Ile Val Trp Tyr Thr Trp His Gly Trp Trp Tyr Ser
            260                 265                 270

Leu Lys Ser Val Val Met Lys Ile Arg Pro Asn Asp Phe Ile Pro Asn
        275                 280                 285

Val Ile
    290